# EUROPEAN PATENT APPLICATION

(11) **EP 4 541 882 A1**
(43) Date of publication of application: **23.04.2025**
(21) Application number: 23823917.2
(22) Date of filing: 13.06.2023
(51) Int. Cl.: C12N 5/071, C12N 5/0783

(54) **THYMIC EPITHELIAL CELL PRODUCTION METHOD**

(30) Priority: 17.06.2022 US 202263353330 P
(71) Applicant: Kyoto University, Kyoto-shi, Kyoto 606-8501 (JP)
(72) Inventor: HAMAZAKI, Yoko, Kyoto-shi, Kyoto 606-8501 (JP); PRETEMER, Yann, Kyoto-shi, Kyoto 606-8501 (JP); GAO, Yuxian, Kyoto-shi, Kyoto 606-8501 (JP)
(74) Representative: Hoffmann Eitle
(86) International application number: PCT/JP2023/021888
(87) International publication number: WO 2023/243627

(57) **Abstract**

The present invention provides a thymic epithelial cell production method, the method including a step for culturing thymic epithelial progenitor cells for a long period of time. The present invention also provides a transplant therapeutic agent that includes thymic epithelial cells produced by the production method according to the present invention. The present invention further provides a T cell production method, the method including a feature of causing thymic epithelial cells produced by the production method according to the present invention to contact hematopoietic stem cells or any cells in the process of differentiation from hematopoietic stem cells to T cells.

## Description

### FIELD

The present invention relates to a method for producing thymic epithelial cells. In particular, the present invention relates to a method for producing thymic epithelial cells, including the step of culturing thymic epithelial progenitors for a long period of time.

### BACKGROUND

The adaptive immune system in a living body ensures the reactivity to theoretically infinite variety of antigens by the presence of a T cell population made up of a certain number of (approximately ten billion) T cells having specificities different from each other. That is to say, even for targets that acquire treatment resistance by changing their antigenicity through mutations (such as cancers and viruses), we can normally recognize and eliminate the targets properly as long as the T cells exhibit their inherent ability to recognize every possible antigen. The ability of T cells to recognize any unknown antigen is conferred by stromal cells called "thymic epithelial cells" in the course of T cell development in the thymus, which is an immune organ. Thymic epithelial cells express the major histocompatibility complex (MHC) unique to each individual, whereby they play a role of: selecting T cells having the ability (MHC restriction) to recognize peptide antigens presented on self-MHC and capable of reacting with a variety of antigens (positive selection); and eliminating T cells (self-reactive T cells) that strongly react with peptides of self-origin (negative selection). Accordingly, by inducing thymic epithelial cells (induced thymic epithelial cells; iTECs) from iPS cells that match the self-MHC and using these iTECs to regenerate T cells from self-hematopoietic stem cells, it is possible to regenerate T cells that do not involve a risk of rejection and have broad reactivity.

Heretofore, methods etc. for inducing differentiation of mouse and human pluripotent stem cells into thymic epithelial progenitors have been reported (for example, NPLs 1 to 5). Conventionally, it has been considered that thymic epithelial progenitors need to be cultured in a three-dimensional environment in order to differentiate them into more mature thymic epithelial cells. Thus, in conventional methods, thymic epithelial progenitors are grafted in a living body of a mouse or the like to induce differentiation of the thymic epithelial progenitors into more mature thymic epithelial cells, and to the best of the inventors' knowledge and belief, inducing differentiation from thymic epithelial progenitors to mature thymic epithelial cells *in vitro* (outside the living body) has not been reported so far.

### [CITATION LIST]

### [NON PATENT LITERATURE]

[NPL 1] Kyle M Loh., et al., Cell Stem Cell. 14(2): 237-52 (2014)
[NPL 2] Gras-Pena R., et al., J Allergy Clin Immunol. 149(5): 1755-1771 (2022)
[NPL 3] Otsuka R., et al., Sci Rep. 10(1): 224 (2020)
[NPL 4] Sun S., et al., Front Immunol. 13: 846281 (2022)
[NPL 5] Ramos S. A., et al., J Allergy Clin Immunol. 149(2): 767-781 (2022)

### SUMMARY

### [TECHNICAL PROBLEM]

With the foregoing in mind, it is an object of the present invention to provide a method for inducing differentiation of thymic epithelial progenitors into mature thymic epithelial cells *in vitro.*

### [SOLUTION TO PROBLEM]

The inventors of the present invention came up with an idea that it might be possible to induce differentiation of pluripotent stem cells into mature thymus epithelial cells by strictly mimicking the signaling event in the developmental process using various factors. Thus, the inventors first made an attempt to identify factors that induce differentiation into, among four pharyngeal pouches present in pharyngeal endoderm (PE), the third pharyngeal pouch from which the thymus anlage is generated. Retinoic acid (RA) is generally known as a factor that forms a concentration gradient, thereby affecting the determination of anterior-posterior patterning in the developmental process of various organs. Thus, the inventors presumed that it would be possible to induce the third pharyngeal pouch by selecting RA as an additive factor and adding RA to a culture medium at an optimal concentration after inducing anterior foregut endoderm (AFE) from definitive endoderm.

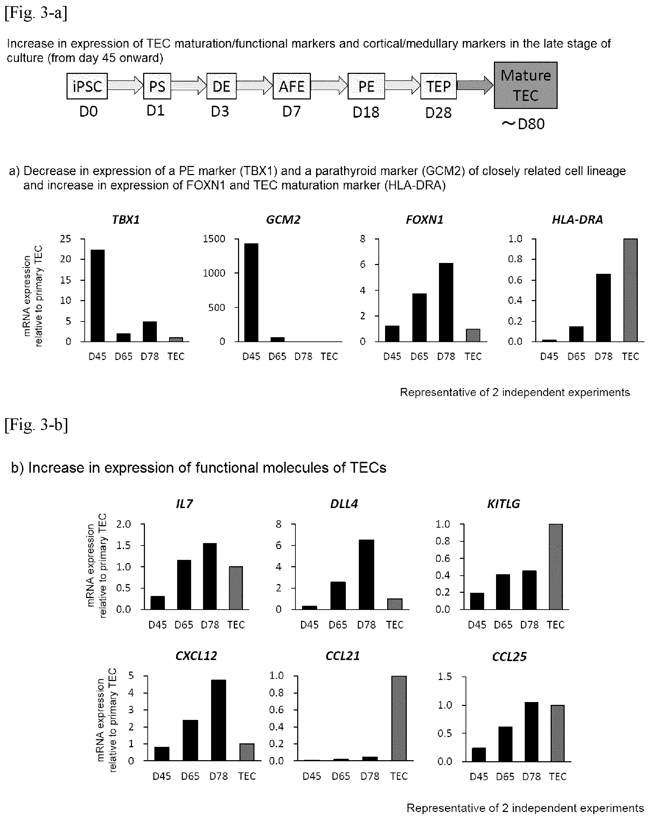

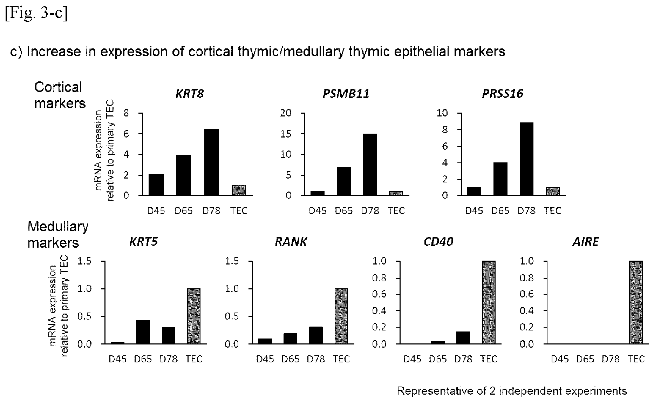

The inventors found out, as a result of their study, that the expression level of Hoxa3, which is selectively expressed in the third and fourth pharyngeal pouches and plays an important role in thymic development, is regulated by the RA concentration. The inventors further searched for factors that strongly induce the expression of FOXN1 after the PE stage, but they did not find such factors. However, as shown in FIG. 3, it was surprisingly found out that, when PE cells were cultured for a long period of time in an environment excluding factors such as RA, FGF-8, and Activin A, which are considered important for inducing differentiation from pluripotent stem cells to thymic epithelial progenitors, the expression level of FOXN1 in the cells increased gradually and at the same time, the expression level of a PE marker TBX1 and the expression level a parathyroid marker GCM2, which is also generated from the third pharyngeal pouch, decreased gradually.

According to these results, it was considered that, by determining an appropriate RA concentration, differentiation into the endoderm of the third pharyngeal pouch could be induced precisely, after which fate determination into the thymic epithelium proceeded autonomously and the cells grew into mature cells over time. These results also suggest that, after the fate determination of the third pharyngeal pouch, thymic development proceeds as a standard pathway and some other stimulus would be necessary in order to induce differentiate into the parathyroid gland. Based on these findings, the inventors conducted further in-depth study, which led to completion of the present invention.

Therefore, the present invention provides the following.
[1] A method for producing thymic epithelial cells, comprising culturing thymic epithelial progenitors for a long period of time.
[2] The method according to [1], wherein the thymic epithelial progenitors are cultured for at least 25 days.
[3] The method according to [1] or [2], wherein the thymic epithelial progenitors are cultured in the absence of at least retinoic acid and FGF-8.
[4] The method according to any one of [1] to [3], wherein the thymic epithelial progenitors are cultured in the absence of at least one factor selected from the group consisting of sonic hedgehog, BMP-4, and noggin.
[5] The method according to any one of [1] to [4], wherein the thymic epithelial progenitors are obtained by culturing pharyngeal endoderm cells in the absence of at least retinoic acid and FGF-8.
[6] The method according to [5], wherein the pharyngeal endoderm cells are obtained by culturing anterior foregut endoderm cells in the presence of retinoic acid.
[7] The method according to [6], wherein the culture is performed in the presence of FGF-8 in addition to the retinoic acid.
[8] The method according to any one of [1] to [7], wherein the culture is performed under feeder-free and/or xeno-free conditions.
[9] The method according to any one of [1] to [8], comprising sorting cells using an expression intensity of FOXN1 as an index.
[10] A thymic epithelial cells obtainable by the method according to any one of [1] to [9].
[11] An agent for transplantation therapy, comprising the thymic epithelial cells according to [10].
[12] Transplantation therapy, comprising administering the thymic epithelial cells according to [10] to a subject.
[13-1] The cells according to [10] for use in transplantation.
[13-2] The cells according to [10] for use in prevention and/or treatment of a disease.
[14-1] Use of the cells according to [10] in the manufacture of an agent for transplantation therapy.
[14-2] Use of the cells according to [10] in the manufacture of a prophylactic medicament and/or treatment medicament for a disease.
[15] A method for producing T cells, comprising bringing the thymic epithelial cells according to
[10] into contact with hematopoietic stem cells or with any cells that are in a differentiation process from hematopoietic stem cells to T cells.
[16] The method according to [15], wherein the thymic epithelial cells and the hematopoietic stem cells are derived from the same individual.
[17] The method according to [15] or [16], wherein the T cells are selected from the group consisting of CD4 and CD8 double-positive cells, CD4 positive cells, and CD8 positive cells.
[18] The method according to any one of [15] to [17], wherein the T cells are a cell population comprising a plurality of T cells that express different TCRs.
[19] T cells obtainable by the method according to any one of [15] to [18].
[20] An agent for immunotherapy, comprising the T cell according to [19].
[21] Immunotherapy comprising administering the T cells according to [19] to a subject.
[22] A method for preventing and/or treating an immunodeficiency disease and/or a cancer, comprising administering the T cells according to [19] to a subject.
[23-1] The cells according to [19] for use in immunotherapy.
[23-2] The cells according to [19] for use in prevention and/or treatment of an immunodeficiency disease and/or a cancer.
[24-1] Use of the cells according to [19] in the manufacture of an agent for immunotherapy.
[24-2] Use of the cells according to [19] in the manufacture of a prophylactic medicament and/or treatment medicament for an immunodeficiency disease and/or a cancer.
[25] A method for producing thymic epithelial progenitors, comprising the steps of:
   (i) culturing anterior foregut endoderm cells in the presence of retinoic acid to induce differentiation into pharyngeal endoderm cells; and
   (ii) culturing the pharyngeal endoderm cells in the absence of at least retinoic acid and FGF-8 to induce differentiation into thymic epithelial progenitors.
[26] The method according to [25], wherein the culture in the step (i) is performed in the presence of FGF-8 in addition to the retinoic acid.
[27] Thymic epithelial progenitors obtainable by the method according to [25] or [26].
[28] An agent for transplantation therapy, comprising the thymic epithelial progenitors according to [27].
[29] Transplantation therapy comprising administering the thymic epithelial progenitors according to [27] to a subject.
[30-1] The cells according to [27] for use in transplantation therapy.
[30-2] The cells according to [27] for use in prevention and/or treatment of a disease.
[31-1] Use of the cells according to [27] in the manufacture of an agent for transplantation therapy.
[31-2] Use of the cells according to [27] in the manufacture of a prophylactic medicament and/or treatment medicament for a disease.

### [ADVANTAGEOUS EFFECTS OF INVENTION]

The present invention enables induction of mature thymic epithelial cells capable of functioning as stromal cells when producing (including "reproducing", the same applies hereinafter) T cells with broad reactivity.

### BRIEF DESCRIPTION OF DRAWINGS

[FIG. 1] FIG. 1 shows increase in expression of marker genes in respective developmental stages in the induction process to TEPs (FOXN1-expressing cells).
[FIG. 2-a] FIG. 2 confirms the expression of FOXN1 using FOXN1 reporter iPS cells: a vector used for establishment of a FOXN1-mCherry reporter iPS cell line (FIG. 2a).

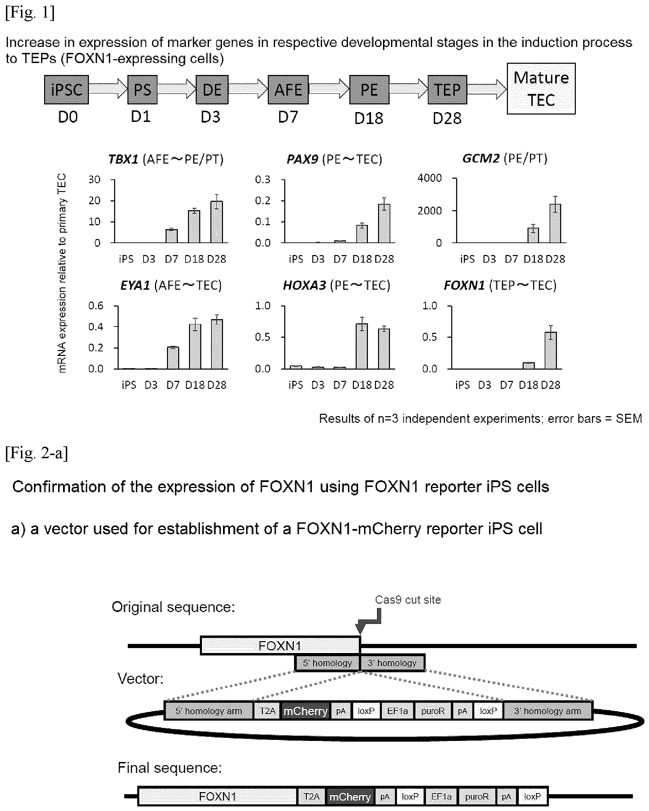

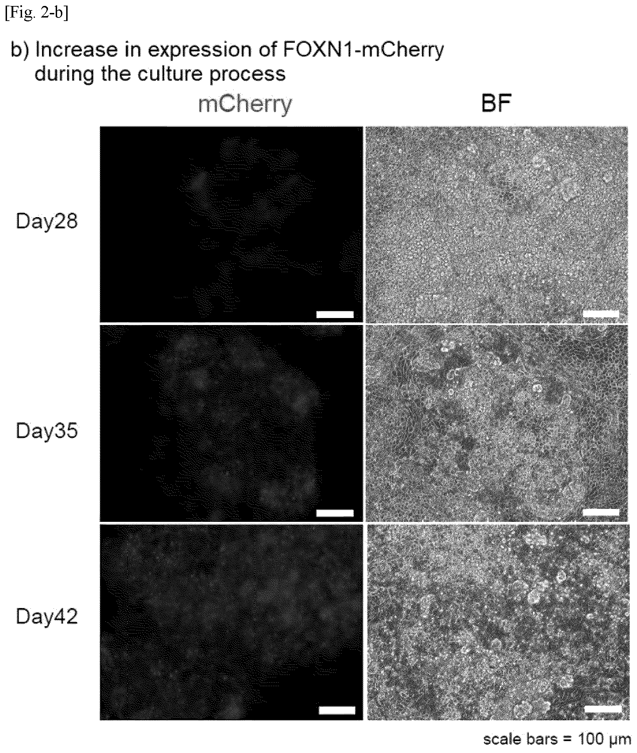

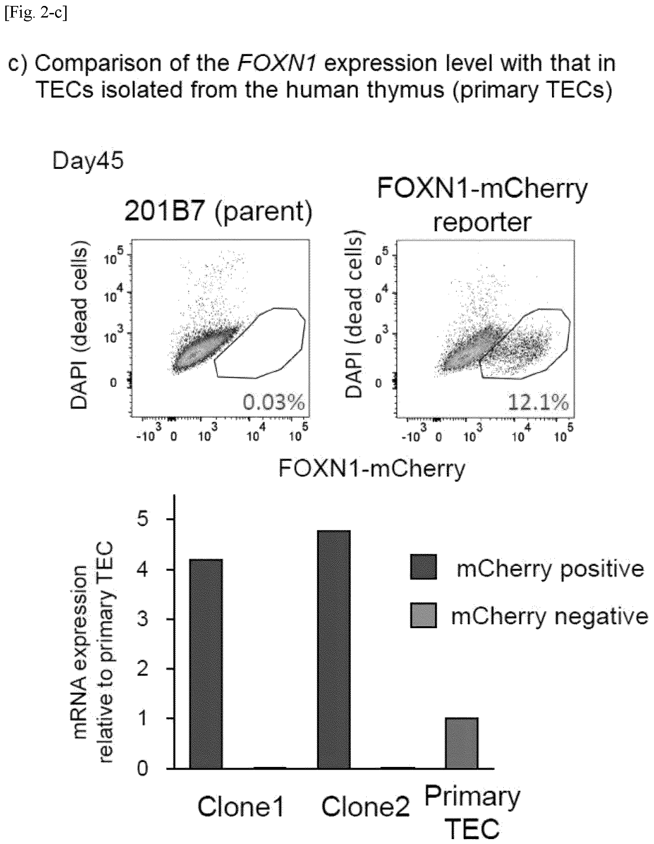

[FIG. 2-b] FIG. 2 confirms the expression of FOXN1 using FOXN1 reporter iPS cells: increase in expression of FOXN1-mCherry during the culture process (FIG. 2b).
[FIG. 2-c] FIG. 2 confirms the expression of FOXN1 using FOXN1 reporter iPS cells: comparison of the FOXN1 expression level with that in TECs isolated from the human thymus (primary TECs) (FIG. 2c).
[FIG. 3-a] FIG. 3 confirms increase in expression of TEC maturation/functional markers and cortical/medullary markers in the late stage of culture (from day 45 onward): decrease in expression of a PE marker (TBX1) and a parathyroid marker (GCM2) of closely related cell lineage and increase in expression of FOXN1 and TEC maturation marker (HLA-DRA) (FIG. 3a).
[FIG. 3-b] FIG. 3 confirms increase in expression of TEC maturation/functional markers and cortical/medullary markers in the late stage of culture (from day 45 onward): increase in expression of functional molecules of TECs (FIG. 3b).
[FIG. 3-c] FIG. 3 confirms increase in expression of TEC maturation/functional markers and cortical/medullary markers in the late stage of culture (from day 45 onward): increase in expression of cortical thymic/medullary thymic epithelial markers (FIG. 3c).

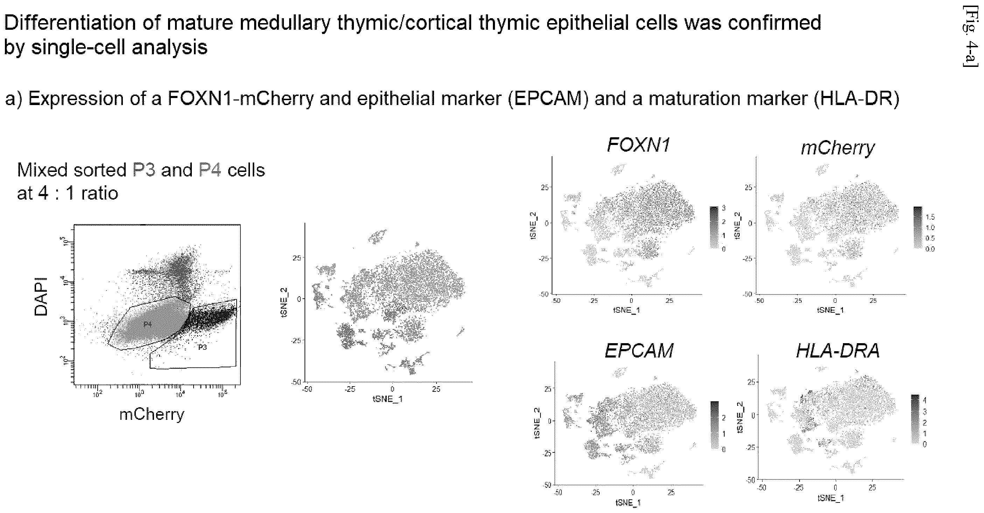

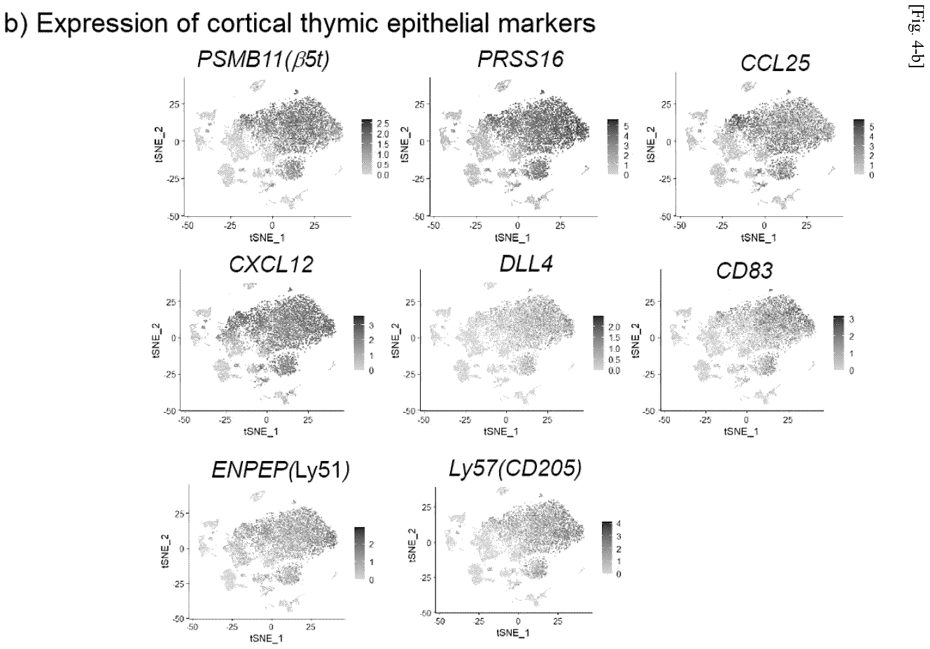

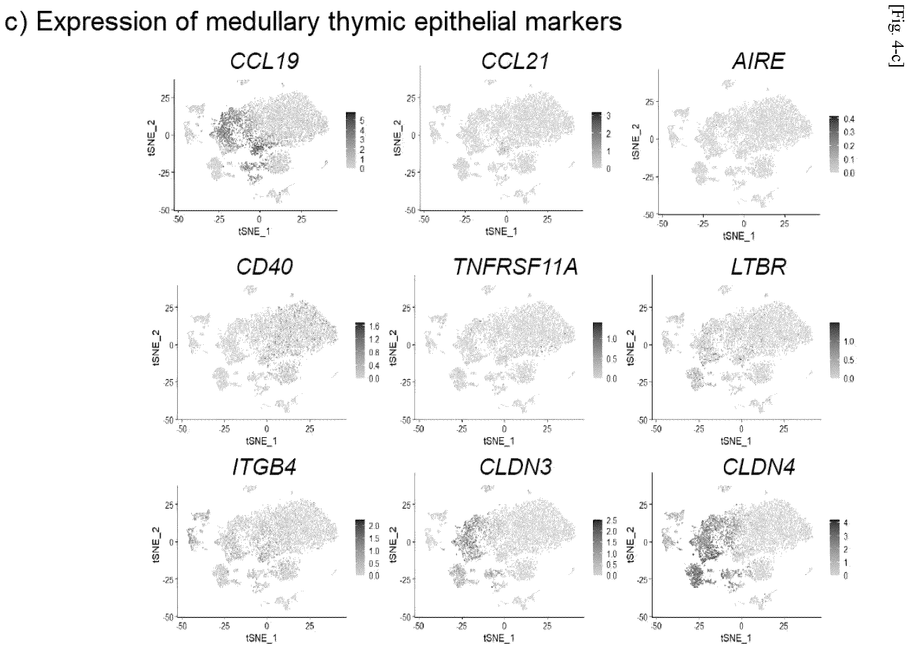

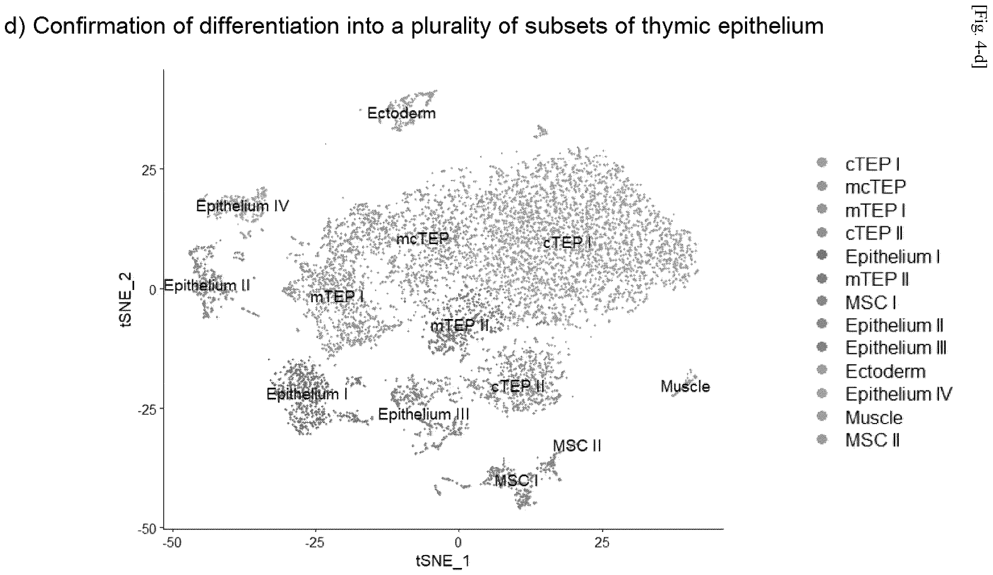

[FIG. 4-a] FIG. 4 confirms differentiation of mature medullary thymic/cortical thymic epithelial cells by single-cell analysis: expression of a FOXN1-mCherry and epithelial marker (EPCAM) and a maturation marker (HLA-DR) (FIG. 4a).
[FIG. 4-b] FIG. 4 confirms differentiation of mature medullary thymic/cortical thymic epithelial cells by single-cell analysis: expression of cortical thymic epithelial markers (FIG. 4b).
[FIG. 4-c] FIG. 4 confirms differentiation of mature medullary thymic/cortical thymic epithelial cells by single-cell analysis: expression of medullary thymic epithelial markers (FIG. 4c).
[FIG. 4-d] FIG. 4 confirms differentiation of mature medullary thymic/cortical thymic epithelial cells by single-cell analysis: confirmation of differentiation into a plurality of subsets of thymic epithelium (FIG. 4d).

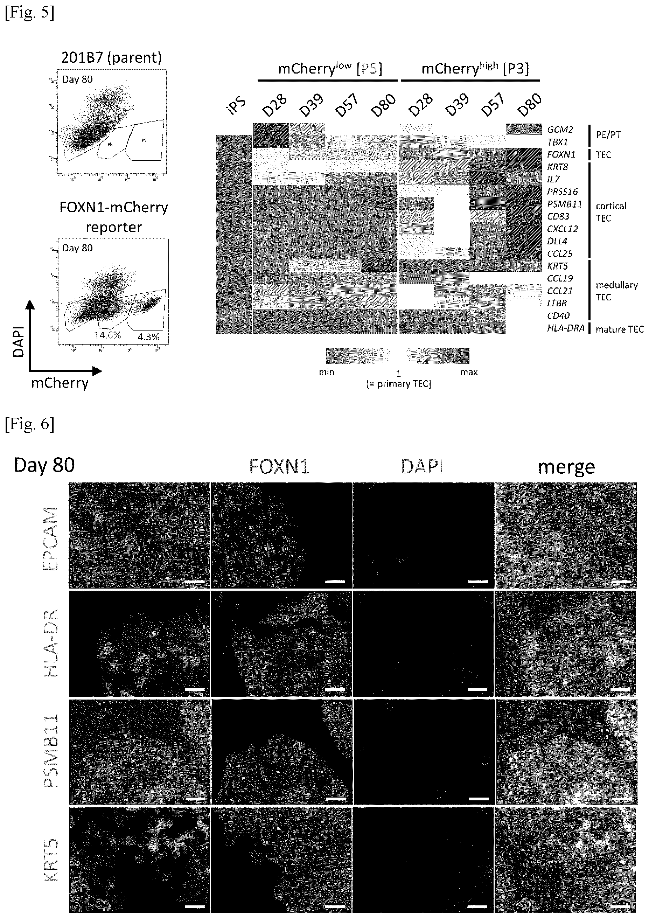

[FIG. 5] FIG. 5 shows, on the left, representative results of n = 6 independent flow cytometry experiments. It was found through observation that a mCherry⁺ population is divided into two different populations, namely, a mCherry^{low} population and a mCherry^{high} population. FIG. 5 shows, on the right, the average value of expression levels of each marker in qPCR experiments (average values of n = 4 independent experiments) in which cells separately sorted from these two cell populations were compared with primary TECs sorted using EPCAM⁺.
[FIG. 6] FIG. 6 confirms differentiation of mature medullary thymic/cortical thymic epithelial cells by immunostaining. Scale bar = 50 µm. FIG. 6 shows representative photographs of n = 5 independent experiments in which FOXN1-mCherry reporters of the 201B7 iPSC line were used. Similar results were obtained when FOXN1-mCherry reporters of the 409B2 and 1383D6 iPSC lines were used.

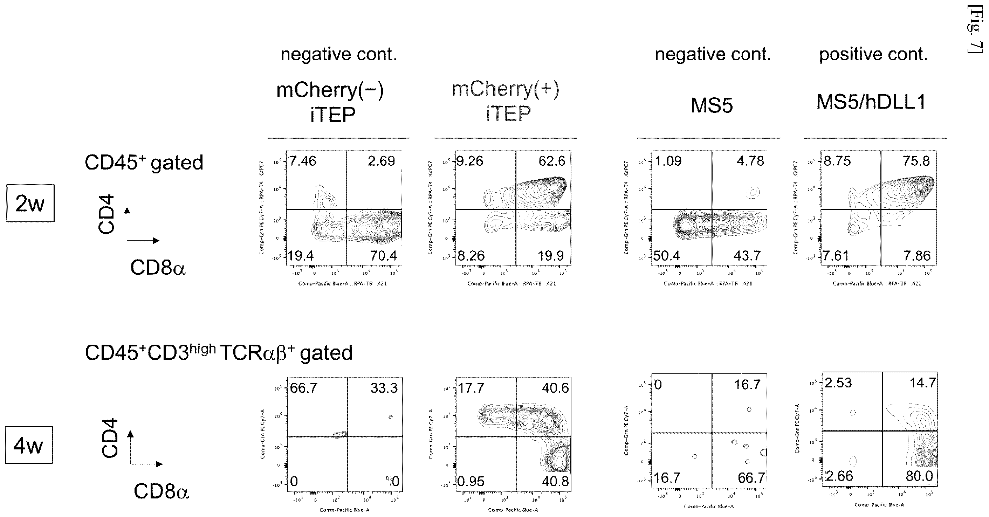

[FIG. 7] FIG. 7 shows the results of generating CD4/CD8 double-positive (DP) and single-positive (SP) thymocytes using iTEP artificial thymic organoid (ATO) culture.

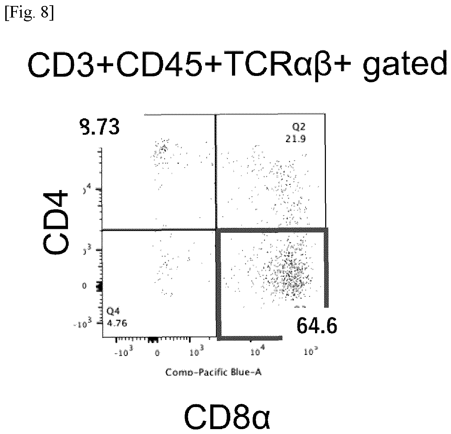

[FIG. 8] FIG. 8 shows the overview of sorting method of organoids for repertoire analysis.

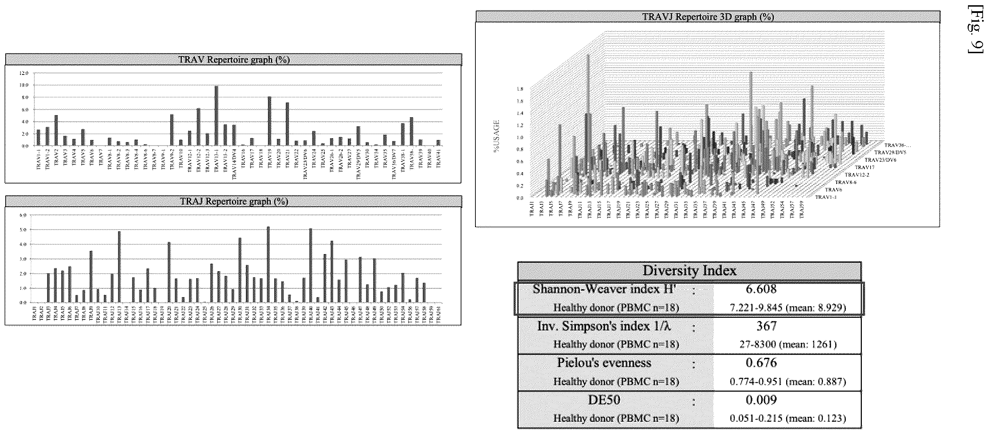

[FIG. 9] FIG. 9 shows the results of repertoire analysis for TCR(T cell receptor)α: a two-dimensional graph showing repertoires of the V (variable) region of TCRα (TRAV) (upper left), a two-dimensional graph showing repertoires of the J (joining) region of TCRα (TRAJ) (lower left), a three-dimensional graph of repertoires of TRAV and TRAJ (upper right), and each diversity index (lower right).

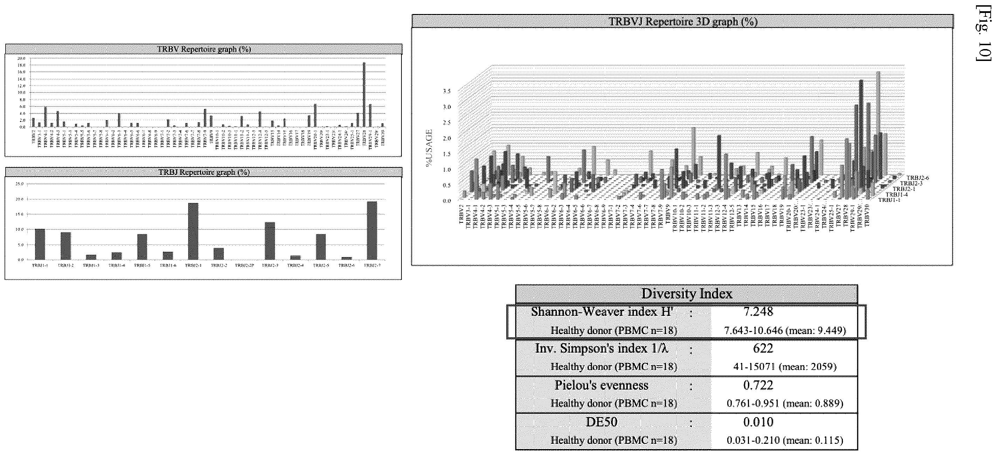

[FIG. 10] FIG. 10 shows the results of repertoire analysis for TCRβ: a two-dimensional graph showing repertoires of the V region of TCRβ (TRBV) (upper left), a two-dimensional graph showing repertoires of the J region of TCRβ (TRBJ) (lower left), a three-dimensional graph of repertoires of TRBV and TRBJ (upper right), and each diversity index (lower right).

### DESCRIPTION OF EMBODIMENTS

As used herein, the singular forms "a", "an" and "the" are intended to include both the singular and plural forms, unless the language explicitly indicates otherwise with words like "only" "single" and/or "one". It will be further understood that the terms "comprises", "comprising", "includes" and/or "including" when used herein, specify the presence of stated features, steps, operations, elements, ideas, and/or components, but do not themselves preclude the presence or addition of one or more other features, steps, operations, elements, components, ideas, and/or groups thereof.

### 1. Thymic epithelial cell production method

The present invention provides a method for producing thymic epithelial cells from thymic epithelial progenitors. Specifically, such a method comprises culturing the thymic epithelial progenitors for a long period of time (the method is also referred to as "the production method of the present invention" hereinafter). The present invention also provides thymic epithelial cells obtainable by the production method of the present invention.

The term "thymic epithelial progenitor (TEP)" as used herein refers to a cell that expresses FOXN1 and has a differentiation potency to a thymic epithelial cell. The term "thymic epithelial cell (TEC)" as used herein refers to a cell that expresses, in addition to FOXN1, at least one thymic epithelial cell marker selected from the group consisting of HLA-DRA (major histocompatibility complex, class II, DR alpha), IL-7 (interleukin 7), KITLG (KIT ligand), CCL21 (chemokine (C-C motif) ligand 21), CCL25 (chemokine (C-C motif) ligand 25), CXCL12 (C-X-C motif chemokine ligand 12), and DLL4 (delta-like canonical notch ligand 4). In the present invention, the thymic epithelial cell preferably expresses at least FOXN1 and HLA-DRA.

In the present specification, the thymic epithelial cell encompasses a cortical thymic epithelial cell (cTEC) and a medullary thymic epithelial cell (mTEC). The term "cortical thymic epithelial cell" as used herein refers to a thymic epithelial cell as defined above, further expressing at least one cortical epithelial marker selected from the group consisting of KRT8 (keratin 8), PSMB11 (proteasome subunit beta 11), and PRSS16 (thymus-specific serine protease). In one embodiment, the cortical thymic epithelial cell expresses at least KRT8 and preferably further expresses PSMB11 and PRSS16. The term "medullary thymic epithelial cell" as used herein refers to a thymic epithelial cell as defined above, further expressing at least one medullary epithelial marker selected from the group consisting of KRT5 (keratin 5), RANK (receptor activator of NF-kB), CD40, and AIRE (autoimmune regulator). In one embodiment, the medullary thymic epithelial cell expresses at least KRT5 and RANK and preferably further expresses CD40.

In the production method of the present invention, the culture period of the thymic epithelial progenitors is typically at least 25 days (e.g., at least 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, (99, 100, 101, 102, 103, 104, 105, 106, 107, 108, 109, or 110 days). The culture period is preferably 35 to 110 days, more preferably 40 to 100 days, and still more preferably 45 to 90 days.

The culture density of the cells is not limited to any particular value as long as the cells can grow. The culture density is typically 1.0 × 10¹ to 1.0 × 10⁶ cells/cm², preferably 1.0 × 10² to 1.0 × 10⁵ cells/cm², more preferably 1.0 × 10³ to 1.0 × 10⁴ cells/cm², and still more preferably 1.0 × 10³ to 3.0 × 10³ cells/cm². Such a cell density is also applicable to each of the steps of inducing differentiation from pluripotent stem cells to thymic epithelial progenitors to be described below.

The production method of the present invention is characterized in that, in the step of culturing thymic epithelial progenitors for a long period of time (e.g., at least 25 days), the thymic epithelial progenitors are cultured typically in a base medium to be described below without adding factors or the like (e.g., factors for Hoxa3 stimulation (specifically, retinoic acid etc.), factors that stimulate the expression of TBX1 (specifically, FGF-8 etc.), factors that stimulate the expression of PAX9 and PAX1 (specifically, sonic hedgehog (Shh) etc.), BMPs, and factors that inhibit BMPs (specifically, noggin etc.)) to the base medium (i.e., in the absence of such factors or the like). Accordingly, the production method of the present invention is characterized in that the thymic epithelial progenitors are cultured in the absence of at least retinoic acid and FGF-8. In one embodiment of the present invention, the production method is also characterized in that the thymic epithelial progenitors are cultured in the absence of at least one factor (preferably all factors) selected from the group consisting of sonic hedgehog, BMP-4, and noggin.

Likewise, the thymic epithelial progenitors to be used in the production method of the present invention can be obtained typically by culturing pharyngeal endoderm cells in a base medium to be described below without adding factors or the like (e.g., factors for Hoxa3 stimulation (specifically, retinoic acid etc.), factors that stimulate the expression of TBX1 (specifically, FGF-8 etc.), factors that stimulate the expression of PAX9 and PAX1 (specifically, sonic hedgehog (Shh) etc.), BMPs, and factors that inhibit BMPs (specifically, noggin etc.)) to the base medium (i.e., in the absence of such factors etc.). Accordingly, in one embodiment of the present invention, the thymic epithelial progenitors to be used in the production method of the present invention are obtained by culturing pharyngeal endoderm cells in the absence of at least retinoic acid and FGF-8. The term "pharyngeal endoderm" as used herein refers to cells that express PAX9 and have a differentiation potency to thymic epithelial progenitors.

In the production method of the present invention, the culture period for inducing the differentiation of the pharyngeal endoderm cells into the thymic epithelial progenitors is not limited to any particular time period as long as desired cells can be obtained, and is typically 5 to 20 days, preferably 7 to 15 days, and more preferably 9 to 12 days.

The pharyngeal endoderm cells to be used in the production method of the present invention can be obtained by culturing anterior foregut endoderm in a base medium to be described below with the addition of retinoic acid to the base medium. As used herein, the term "anterior foregut endoderm" refers to cells that express SOX2 and FOXA2 and have a differentiation potency to pharyngeal endoderm. In the present invention, the term "retinoic acid" typically refers to all-trans-3,7-dimethyl-9-(2,6,6-trimethyl-1-cyclohexene-1-yl)-2,4,6,8-nonatetraenoic acid (Cas No: 302-79-4), and shall be construed to encompass "salts of retinoic acid" and "precursors of retinoic acid". Examples of the salts of retinoic acid include, but are not limited to, sodium retinoate, potassium retinoate, and calcium retinoate. Examples of the precursors of retinoic acid include, but are not limited to, β-carotene, retinol esters, retinol, and retinal.

The concentration of the retinoic acid in the medium is not limited to any particular value as long as desired cells can be obtained. When the retinoic acid is all-trans-3,7-dimethyl-9-(2,6,6-trimethyl-1-cyclohexene-1-yl)-2,4,6,8-nonatetraenoic acid or a salt thereof, the concentration of the retinoic acid is typically 10 nM to 2 µM, preferably 50 nM to 600 nM, and more preferably 100 nM to 300 nM, for example. When other retinoic acids are used, the concentration thereof can be determined as appropriate.

The medium for inducing differentiation of anterior foregut endoderm cells into pharyngeal endoderm cells may be supplemented with FGF-8 in addition to the above-described retinoic acid. The concentration of FGF-8 in the medium is not limited to any particular value as long as desired cells are obtained, and typically is, for example, 1 ng/ml to 500 ng/ml, preferably 10 ng/ml to 200 ng/ml, and more preferably 25 ng/ml to 100 ng/ml.

In the production method of the present invention, the culture period for inducing the differentiation of anterior foregut endoderm cells into pharyngeal endoderm cells is not limited to any particular time period as long as desired cells can be obtained, and is typically 4 to 15 days, preferably 6 to 14 days, and more preferably 8 to 13 days.

The anterior foregut endoderm cells to be used in the production method of the present invention may be prepared from (human) pluripotent stem cells by a method known per se (e.g., NPL 1). In one example, first, (human) pluripotent stem cells are cultured in a base medium to be described below, supplemented with Activin A, PI-103, and CHIR to induce differentiation of the pluripotent stem cells into the primitive streak (PS). Next, the primitive streak is cultured in a base medium to be described below, supplemented with Activin A and LDN to induce differentiation of the primitive streak into definitive endoderm (DE). The definitive endoderm may be further cultured in a base medium to be described below, supplemented with LDN and A83-01 to induce differentiation of the definitive endoderm into anterior foregut endoderm cells (AFE).

As used herein, the term "pluripotent stem cells" refers to stem cells that can differentiate into tissue or cells having various different forms or functions in the body, and that can also differentiate into cells of any lineages of the three germ layers (endoderm, mesoderm and ectoderm). Examples of pluripotent stem cells to be used for the present invention include induced pluripotent stem cells (iPS cells), embryonic stem cells (ES cells), embryonic stem cells from cloned embryos obtained by nuclear transfer (nuclear transfer Embryonic stem cells; ntES cells), multipotent germline stem cells (mGS cells) and embryonic germ cells (EG cells), of which iPS cells (and especially human iPS cells) are preferred. When the pluripotent stem cells are ES cells or arbitrary cells derived from human embryos, the cells may be obtained by destruction of the embryo or they may be obtained without destruction of the embryo, but preferably they are cells obtained without destruction of the embryo.

ES cells are stem cells having pluripotency and auto-replicating proliferation potency, established from the inner cell mass of an early embryo (such as the blastocyst) of a mammal such as a human or mouse. ES cells were discovered in mice in 1981 (M.J. Evans and M.H. Kaufman (1981), Nature 292:154-156), and ES cell lines were later established in primates including humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cells can be established by extracting the inner cell mass from the blastocyst of a fertilized egg of a target animal and culturing the inner cell mass on a fibroblast feeder. Alternatively, ES cells can be established using a single embryo blastomere alone during the cleavage stage before the blastocyst stage (Chung Y. et al. (2008), Cell Stem Cell 2: 113-117), or they can be established using a developmentally arrested embryo (Zhang X. et al. (2006), Stem Cells 24: 2669-2676.).

ntES cells are ES cells derived from a clone embryo prepared by nuclear transfer, and they have approximately the same properties as fertilized egg-derived ES cells (Wakayama T. et al. (2001), Science, 292:740-743; S. Wakayama et al. (2005), Biol. Reprod., 72:932-936; Byrne J. et al. (2007), Nature, 450:497-502). In other words, ntES (nuclear transfer ES) cells are ES cells established from the inner cell mass of a cloned embryo-derived blastocyst obtained by exchanging an unfertilized egg nucleus with a somatic cell nucleus. Combinations of nuclear transfer (Cibelli J.B. et al. (1998), Nature Biotechnol., 16:642-646) and ES cell preparation techniques (described above) are used to prepare ntES cells (Wakayama, S. (2008), Jikken Igaku, Vol. 26, No. 5 (special edition), pp. 47-52). For nuclear transfer, a somatic cell nucleus may be implanted into a mammalian enucleated unfertilized egg and cultured for several hours for reprogramming.

The ES cell line used for the present invention may be mouse ES cells, and for example, the different mouse ES cell lines established by the inGenious Targeting Laboratory, Riken (Riken Research Institute), etc., may be used, or for human ES cell lines, the different human ES cell lines established by the University of Wisconsin, NIH, Riken, Kyoto University, the National Center for Child Health and Development, Cellartis, etc., for example, may be used. Specific examples of human ES cell lines include CHB-1 to CHB-12 lines, RUES1 line, RUES2 line and HUES1 to HUES28 lines distributed by ESI Bio Co., H1 and H9 lines distributed by WiCell Research, KhES-1, KhES-2, KhES-3, KhES-4, KhES-5, SSES1, SSES2 and SSES3 distributed by Riken, and so on.

iPS cells are cells obtained by reprogramming of mammalian somatic cells or undifferentiated stem cells by introduction of specific factors (nuclear reprogramming factors). A large number of iPS cells currently exist, among which there may be used iPSCs established by introduction of the 4 factors Oct3/4·Sox2·Klf4·c-Myc into mouse fibroblasts by Yamanaka et al. (Takahashi K, Yamanaka S., Cell, (2006) 126: 663-676), iPSCs derived from human cells established by introduction of the same 4 factors into human fibroblasts (Takahashi K, Yamanaka S., et al. Cell, (2007) 131:861-872.), Nanog-iPSCs established by selecting of Nanog expression markers after introduction of the 4 factors (Okita, K., Ichisaka, T. and Yamanaka, S. (2007). Nature 448, 313-317.), iPSCs produced by methods without c-Myc (Nakagawa M, Yamanaka S., et al. Nature Biotechnology, (2008) 26, 101-106), iPSCs established by introduction of 6 factors by a virus-free method (Okita K et al. Nat. Methods 2011 May; 8(5):409-12, Okita K et al. Stem Cells. 31(3):458-66.), and so on. The induced pluripotent stem cells established by introduction of the 4 factors OCT3/4·SOX2·NANOG·L1N28, created by Thomson et al. (Yu J., Thomson JA. et al., Science (2007) 318: 1917-1920), the induced pluripotent stem cells created by Daley et al. (Park IH, Daley GQ. et al., Nature (2007) 451:141-146) and the induced pluripotent stem cells created by Sakurata et al. (Japanese Unexamined Patent Publication No. 2008-307007), etc., may also be used.

In addition, any induced pluripotent stem cells publicly known in the field as described in published journal (for example, Shi Y., Ding S., et al., Cell Stem Cell, (2008) Vol. 3, Issue 5, 568-574;, Kim JB., Scholer HR., et al., Nature, (2008) 454, 646-650; Huangfu D., Melton, DA., et al., Nature Biotechnology, (2008) 26, No 7, 795-797), or patents (for example, Japanese Unexamined Patent Publication No. 2008-307007, Japanese Unexamined Patent Publication No. 2008-283972, US2008-2336610, US2009-047263, WO2007-069666, WO2008-118220, WO2008-124133, WO2008-151058, WO2009-006930, WO2009-006997 and WO2009-007852), may also be used.

Induced pluripotent stem cell lines that are iPSC lines established by NIH, Riken, Kyoto University, etc., for example, may be used as well. Examples include human iPSC lines such as HiPS-RIKEN-1A, HiPS-RIKEN-2A, HiPS-RIKEN-12A, Nips-B2, etc., by Riken, 253G1, 253G4, 1201C1, 1205D1, 1210B2, 1383D2, 1383D6, 201B7, 409B2, 454E2, 606A1, 610B1, 648A1, 1231A3, FfI-01s04, and QHJI01s04 by Kyoto University, and TC-1133HKK_05G and TC-1133HKK_06E by Lonza, and iPSC strains obtained by genetically modifying the above-mentioned iPSC strains, and so on.

mGS cells are pluripotent stem cells derived from the testes, and they serve as a source for spermatogenesis. Similar to ES cells, these cells can also be induced to differentiate to cells of various cell series, and have properties that allow creation of a chimeric mouse when they are grafted into a mouse blastocyst, for example (Kanatsu-Shinohara M. et al. (2003) Biol. Reprod., 69:612-616; Shinohara K. et al. (2004), Cell, 119:1001-1012). The mGS cells are also capable of auto-replication in culture medium containing glial cell line-derived neurotrophic factor (GDNF), and their repeated subculturing under culturing conditions similar to those of ES cells allows germline stem cells to be obtained (Takebayashi, M. et al. (2008), Jikken Igaku, Vol. 26, No. 5 (Special Edition) pp. 41-46, Yodosha (Tokyo, Japan)).

EG cells are cells with pluripotency similar to ES cells, being established from embryonic primordial germ cells. The EG cells can be established by culturing primordial germ cells in the presence of substances such as LIF, bFGF and stem cell factors (Matsui Y. et al. (1992), Cell, 70:841-847; J. L. Resnick et al. (1992), Nature, 359:550-551).

The source species of the pluripotent stem cells is not particularly restricted, and for example, the cells may be from a rodent such as a rat, mouse, hamster or guinea pig, a lagomorph such as a rabbit, an ungulate such as a pig, cow, goat or sheep, a dog, a feline such as a cat, or a primate such as a human, monkey, rhesus monkey, marmoset, orangutan or chimpanzee. The preferred source species is human.

The term "cells" as used herein includes "cell populations", unless otherwise specified. A cell population may be composed of a single type of cells or two or more different types of cells.

In the production method of the present invention, all or at least one of the steps involved in the production of thymic epithelial cells may be performed under feeder-free and/or xeno-free conditions. Preferably, all the steps involved in the production of thymic epithelial cells are performed under feeder-free and xeno-free conditions. As used herein, the term "feeder-free" refers to a medium or culturing conditions free of auxiliary cells (i.e., feeder cells) that are different in type from cells to be cultured and used for adjusting culturing conditions for the cells to be cultured. The term "xeno-free" refers to a medium or culturing conditions free of components derived from organisms of biological species that are different from that of cells to be cultured.

Examples of a base medium that can be used in the production method of the present invention include, but are not limited to: a RPMI-1640 medium, Eagle's MEM (EMEM), Dulbecco's modified MEM (DMEM), Glasgow's MEM (GMEM), α-MEM, 199 medium, IMDM, Hybridoma Serum free medium, KnockOut^{™} DMEM (KO DMEM), Advanced^{™} media (e.g., Advanced MEM, Advanced RPMI, and Advanced DMEM/F-12), Chemically Defined Hybridoma Serum free medium, Ham's Medium F-12, Ham's Medium F-10, Ham's Medium F12K, DMEM/F-12, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's Medium (e.g., Waymouth's MB752/1), CMRL medium (e.g., CMRL-1066), Williams' medium E, Brinster's BMOC-3 Medium, E8 Medium, StemPro 34, and MesenPRO RS (all Thermo Fisher Scientific); ReproFF2, Primate ES Cell Medium, and ReproStem (all ReproCELL Incorporated); Procul AD (ROHTO Pharmaceutical Co., Ltd.); MSCBM-CD and MSCGM-CD (both Lonza); EX-CELL 302 medium (SAFC) or EX-CELL-CD-CHO (SAFC); ReproMed^{™} iPSC Medium (ReproCELL Incorporated); Cellartis MSC Xeno-Free Culture Medium (Takara Bio Inc.); TESR-E8 (Veritas Corporation); StemFit (registered trademark) AK02N, AK03N, and Basic 03 (all Ajinomoto Co., Inc.); CTS (registered trademark) KnockOut SR XenoFree Medium (Gibco); mTeSR1 medium and TeSR1 medium (Stem Cell Technologies); Iscove's modified Dulbecco's medium (GE HealthCare); CDM2; CDM3; and mixtures thereof.

The base medium may be supplemented with biologically active substances, nutritional factors, etc. required for survival or growth of cells, when necessary. Such medium additives may be added to the medium beforehand or may be added during cell culture. They may be added in any form, e.g., in the form of a solution or a mixture of two or more solutions, and may be added continuously or intermittently.

Examples of the biologically active substances include insulin, IGF-1, transferrin, albumin, coenzyme Q10, various types of cytokines (interleukins (IL-2, IL-7, IL-15, etc.), stem cell factors (SCFs), activin, etc.), various types of hormones, and various types of growth factors (leukemia inhibitory factors (LIFs), basic fibroblast growth factor (bFGFs), TGF-β, etc.). Examples of the nutritional factors include sugars, amino acids, vitamins, hydrolysates, and lipids. Examples of the sugars include glucose, mannose, and fructose. One type of sugar may be used, or two or more types of sugars may be used in combination. Examples of the amino acids include L-alanine, L-arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, and L-valine. One type of amino acid may be used, or two or more types of amino acids may be used in combination. Examples of the vitamins include D-biotin, D-pantothenic acid, choline, folic acid, myo-inositol, niacinamide, pyridoxal, riboflavin, thiamine, cyanocobalamin, and DL-α-tocopherol. One type of vitamin may be used, or two or more types of vitamins may be used in combination. Examples of the hydrolysates include those obtained by hydrolyzing soybeans, wheat, rice, peas, corn, cottonseeds, and yeast extracts. Examples of the lipids include cholesterol, linoleic acid, and linolenic acid. Examples of polysaccharides include gellan gum, deacylated gellan gum, methylcellulose, carboxymethylcellulose, hydroxyethylcellulose, hydroxypropylcellulose, carboxymethylamylose, xanthan gum, alginic acid, carrageenan, diutan gum, and locust bean gum.

The medium may be further supplemented with an antibiotic such as kanamycin, streptomycin, penicillin, or hygromycin, when necessary. When an acidic substance such as sialic acid is added to the medium, it is desirable to adjust the pH of the medium to a value in a neutral range suitable for cell growth, namely, pH 5 to 9 and preferably pH 6 to 8.

The medium to be used in the production method of the present invention may be a medium containing a serum (e.g., fetal bovine serum (FBS), human serum, or horse serum) or a serum-free medium. From the viewpoint of preventing contamination with components derived from different animal species, it is preferable that the medium does not contain a serum or that a serum to be used is derived from the same animal species as the source animal species of the cells to be cultured. The serum-free medium as used herein refers to a medium that does not contain unadjusted or unpurified serum. The serum-free medium may contain purified blood-derived components or animal tissue-derived components (e.g., growth factors).

Examples of a serum replacement include albumin, albumin replacements such as lipid-rich albumin and recombinant albumin, plant starch, dextran, protein hydrolysates, transferrin or other iron transporters, fatty acid, insulin, collagen precursors, trace elements, 2-mercaptoethanol, 3'-thioglycerol, and equivalents thereof. Specific examples of the serum replacement include those prepared according to the method described in International Patent Publication No. WO98/30679 and commercially available serum replacements such as Knockout Serum Replacement [KSR] (Life Technologies), Chemically-defined Lipid concentrated (Life Technologies), and L-alanine-L-glutamine dipeptide (e.g., Glutamax (Life Technologies)). Examples of biological factors include platelet-rich plasma (PRP) and components contained in a culture supernatant of human mesenchymal stem cells.

In the production method of the present invention, the culture method may be either adhesion culture or suspension culture, of which adhesion culture is preferable from the viewpoint of operability and avoiding differentiation into undesired lineages. A culture vessel used for adhesion culture may be, for example, a culture vessel with its surface having been subjected to an artificial treatment to improve adhesion with cells (e.g., a coating treatment with, for example, an extracellular matrix such as a basement membrane preparation, fibronectin, laminin or its fragments, entactin, collagen, gelatin, Synthemax, or vitronectin or with a polymer such as polylysine or polyornithine, or surface processing such as a positive charge treatment).

Laminin or its fragments to be used for the production method of the present invention include laminin-111 or its fragment containing the E8 domain, laminin-211 or its fragment containing the E8 domain (e.g. iMatrix-211), laminin-121 or its fragment containing the E8 domain, laminin-221 or its fragment containing the E8 domain, laminin-332 or its fragment containing the E8 domain, laminin-3A11 or its fragment containing the E8 domain, laminin-411 or its fragment containing the E8 domain (e.g. iMatrix-411), laminin-421 or its fragment containing the E8 domain, laminin-511 or its fragment containing the E8 domain (e.g. iMatrix-511, iMatrix-511 silk), laminin-521 or its fragment containing the E8 domain, laminin-213 or its fragment containing the E8 domain, laminin-423 or its fragment containing the E8 domain, laminin-523 or its fragment containing the E8 domain, laminin-212/222 or its fragment containing the E8 domain, laminin-522 or its fragment containing the E8 domain, and so on.

The culture vessel used for suspension culture is not particularly restricted so long as it allows "suspension culturing", and may be determined as appropriate by a person skilled in the art. Examples of such culture vessels include a flask, tissue culture flask, dish, petri dish, tissue culture dish, multidish, microplate, microwell plate, micropore, multiplate, multiwell plate, chamber slide, schale, tube, tray, culture bag or roller bottle. A bioreactor is another example of a container for suspension culture. These culture vessels are preferably non-adhesive to cells to allow suspension culture. The non-cell-adhesive culture vessel used may be one where the surface of the culture vessel is not artificially treated to improve adhesion with cells (for example, coating treatment with extracellular matrix).

The culturing temperature in the production method of the present invention is not particularly restricted but may be about 30°C to 40°C and preferably about 37°C, with the culturing being carried out in a CO₂-containing air atmosphere with a CO₂ concentration of preferably about 2% to 5%.

Further, Examples to be described below suggested that a mCherry^{high} population, which represents a FOXN1 high-expressing cell population, mainly contains mature cTECs and few mTECs, whereas a mCherry^{low} population, which represents a FOXN1 low-expressing cell population, contains a mixture of immature mTECs and presumably bipotent progenitors. Thus, by using the expression intensity of FOXN1 as an index, it is possible to obtain cell populations with different cell compositions. Therefore, the production method of the present invention may comprise sorting (which is interchangeable with the term "isolating", "enriching", or "purifying") cells from a cell population obtained by the production method of the present invention using the expression intensity of FOXN1 as an index. Cell sorting may be performed using any known method as appropriate. For example, cells can be sorted by flow cytometry using the intensity of fluorescent protein expression, which reflects the FOXN1 expression, as an index. More specifically, FOXN1 high-expressing cells or FOXN1 low-expressing cells can be sorted from a cell population by using, for example: cells in which a construct including a fluorescent protein-coding gene linked under the control of a promoter of the FOXN1 gene has been introduced; cells in which the FOXN1 gene on one of alleles has been substituted with a gene encoding a fluorescent protein or a fusion protein composed of FOXN1 and a fluorescent protein by genome editing using a CRISPR/Cas system or the like; or cells in which the FOXN1 gene and a gene encoding a fluorescent protein have been incorporated into the genome via a sequence that enables polycistronic expression by genome editing. Examples of the sequence that enables polycistronic expression include 2A sequences (e.g., a 2A sequence derived from foot-and-mouth disease virus (FMDV) (F2A), 2A sequence derived from equine rhinitis A virus (ERAV) (E2A), 2A sequence derived from Porcine teschovirus (PTV-1) (P2A), and 2A sequence derived from Thosea asigna virus (TaV) (T2A)) (PLoS ONE, 3:e2532, 2008, Stem Cells 25, 1707, 2007, etc.) and internal ribosome entry sites (IRESs) (U.S. Patent No. 4,937,190). From the viewpoint of uniform expression, 2A sequences are preferable.

The description that a gene is "expressed" or "positive", unless otherwise specified, is used herein in the sense that includes at least "production of mRNA encoded by the gene", and preferably also "production of a protein encoded by the mRNA". Therefore, the gene may be said to be expressed if production of mRNA encoded by the gene is detected, at least by the method described in the Examples below (RT-qPCR).

As used herein, the term "positive" includes the concepts of high expression (also called "high positive") and low expression (also called "low positive"). High expression may be referred to as "high" or "bright." For example, FOXN1 high-expressing cells may be referred to as "FOXN1^{high}" cells or "FOXN1^{bright}" cells. Low expression may be referred to as "low" or "dim." For example, FOXN1 low-expressing cells may be referred to as "FOXN1^{low}" cells or "FOXN1^{dim}" cells. High positivity and low positivity can be judged based on a chart obtained by flow cytometry. The position in the chart may vary depending on the voltage setting and sensitivity setting of the device, and the antibody clone used, the staining conditions and the dye used, etc., but a person skilled in the art can appropriately draw lines so as not to separate cell populations that are to be treated as a single group in the obtained chart.

### 2. Thymic epithelial progenitor production method

The present invention also provides a method for producing thymic epithelial progenitors from anterior foregut endoderm cells. Specifically, such a method comprises the steps of: i) culturing anterior foregut endoderm cells in the presence of retinoic acid to induce differentiation into pharyngeal endoderm cells; and (ii) culturing the pharyngeal endoderm cell in the absence of at least retinoic acid and FGF-8 to induce differentiation into thymic epithelial progenitors. The above-described progenitor production method may be adapted such that the culture in the step (i) is performed in the presence of FGF-8 in addition to the retinoic acid. The present invention also provides thymic epithelial progenitors obtainable by the production method of the present invention. Regarding specific culture methods and the like to be used in the thymic epithelial progenitor cell production method of the present invention, descriptions on culture methods and the like provided in the above section "1. Thymic epithelial cell production method" are all incorporated in the present section.

### 3. Agent for cell transplantation therapy

Thymic epithelial cells obtained by the production method of the present invention (hereinafter also referred to as "thymic epithelial cells of the present invention") are more mature than cells obtained by conventional methods from the viewpoint of maturation markers (e.g., HLA-DRA) and functional molecules (e.g., IL7, DLL4, KITLG, CXCL12, CCL21, and CCL25) in these cells as described above, and thus are capable of sufficiently serving as stromal cells in production of T cells with broad reactivity. Accordingly, the thymic epithelial cells of the present invention can be suitably used in cell transplantation therapy. Therefore, viewed from another aspect, the present invention provides an agent for cell transplantation therapy, comprising the thymic epithelial cells of the present invention (hereinafter also referred to as "agent 1 for cell transplantation therapy according to the present invention"). Viewed from still another aspect, the present invention provides transplantation therapy including administering the thymic epithelial cells of the present invention to a subject. Alternatively, the thymic epithelial progenitors obtained by the production method of the present invention (hereinafter also referred to as "thymic epithelial progenitors of the present invention") may also be used in cell transplantation therapy since they are also capable of sufficiently serving as stromal cells in production of T cells with broad reactivity. Therefore, viewed from still another aspect, the present invention provides an agent for cell transplantation therapy, comprising the thymic epithelial progenitors of the present invention (hereinafter also referred to as "agent 2 for cell transplantation therapy according to the present invention"). Hereinafter, the term "agent for cell transplantation therapy according to the present invention" may also be used as a collective term for the agents 1 and 2 for cell transplantation therapy according to the present invention. Viewed from still another aspect, the present invention provides transplantation therapy including administering the thymic epithelial progenitors of the present invention to a subject. The subject to which the above-described cells or agent for cell transplantation therapy is administered may be a mammal, which specifically is, for example, a mouse, rat, hamster, rabbit, cat, dog, cow, sheep, monkey, and human, and preferably is a human.

The thymus, which is an organ that produces T cells with broad reactivity, starts to shrink after adolescence and its function deteriorates rapidly. This is one of the causes of age-related decline in immune function. The decline or loss of the thymus function caused by chemotherapy, radiotherapy, or the like is considered highly relevant to, for example, the decline in immune function, the increased frequency of development of autoimmune diseases, and a phenomenon such as delayed T cell recovery after bone marrow transplantation. The inventors of the present invention previously reported that grafting medullary thymic epithelial stem cells into mice suffering from defective medulla formation and thus having a risk of development of autoimmune diseases ensures continuous supply of normal medullary epithelial cells nearly throughout life of each individual mouse, whereby the development of autoimmune diseases is suppressed almost completely (Sekai M. et al. Immunity. 41(5):753-761 (2014)). Also, it has been reported that grafting thymic epithelial-like cells generated from mouse iPS cells into mice with a normal immune system prolongs the survival of skin grafts and regulates the transplant immune response (Otsuka R. et al., Sci Rep. 11: 12857 (2021)). Accordingly, the agent for cell transplantation therapy according to the present invention can be used, for example, for treatment of diseases of the thymus, decline in immune function (for example, age-related decline in immune function), or autoimmune diseases, for promoting T cell recovery after bone marrow transplantation, or for regulating transplant immune response (for example, for preventing rejection and graft-versus-host disease), but the use of the agent for cell transplantation therapy according to the present invention is not limited thereto. Examples of the autoimmune diseases include Basedow's disease, rheumatoid arthritis, Hashimoto's thyroiditis, type 1 diabetes, systemic lupus erythematosus, vasculitis, Addison's disease, polymyositis, dermatomyositis, psoriasis, Sjogren's syndrome, systemic scleroderma, and glomerulonephritis. Alternatively, the agent for cell transplantation therapy according to the present invention can be used in a method for preventing and/or treating diseases associated with a decrease of T cells or declined function of T cells (for example, immunodeficiency diseases and cancers) to be described below.

### 4. T cell production method

As described above, the thymic epithelial cells and thymic epithelial progenitors of the present invention are capable of sufficiently serving as stromal cells in production of T cells with broad reactivity. Accordingly, the present invention also provides a method for producing T cells using the thymic epithelial cells and/or thymic epithelial progenitors of the present invention (such a method is hereinafter also referred to as "T cell production method of the present invention"). Specifically, such a method comprises bringing the thymic epithelial cells and/or thymic epithelial progenitors of the present invention into contact with hematopoietic stem cells or any cells that are in a differentiation process from hematopoietic stem cells to T cells (typically co-culturing these cells). Examples of the cells that are in a differentiation process from hematopoietic stem cells to T cells include hematopoietic progenitors, lymphoid stem cells, and T cell progenitors (e.g., DN1 cells, DN2 cells, DN3 cells, DN4 cells, and DP cells). In the T cell production method of the present invention, for example, by inducing thymic epithelial cells (iTECs) from iPS cells that match the MHC of a particular individual and using the thus-induced iTECs to produce T cells from hematopoietic stem cells of the individual, it becomes possible to regenerate T cells that do not involve a risk of rejection and have broad reactivity.

T cells obtained by the above-described T cell production method (hereinafter also referred to as "T cells of the present invention") can be suitably used for immunotherapy such as T cell transplantation therapy. Therefore, viewed from still another aspect, the present invention provides an agent for immunotherapy, such as an agent for transplantation therapy, comprising the T cells of the present invention. Examples of the T cells of the present invention include CD4 and CD8 double-positive cells, CD4 positive cells (CD4 SP cells), CD8 positive cells (CD8 SP cells), and cell populations comprising two or more types of these cells. The agent for immunotherapy according to the present invention may comprise the thymic epithelial cells and/or thymic epithelial progenitors of the present invention. Viewed from still another aspect, the present invention provides immunotherapy including administering the T cells of the present invention to a subject. The immunotherapy may comprise administering the thymic epithelial cells and/or thymic epithelial progenitors of the present invention at the same time with or at a different timing from the administration of the T cells. This immunotherapy can be used as a method for preventing and/or treating diseases in which T cell reduction or decline in T cell functions is involved (such as primary or secondary immunodeficiency diseases, autoimmune diseases, and cancers, for example). Regulatory T cells, which is one type of CD4 positive T cells, can also be used to suppress adverse immune responses such as autoimmune diseases and transplantation immune responses. The subject to which the above-described cells or agent for immunotherapy is administered may be a mammal, which specifically is, for example, a mouse, rat, hamster, rabbit, cat, dog, cow, sheep, monkey, and human, and preferably is a human.

Immunodeficiency diseases or autoimmune diseases to which the immunotherapy is applicable are typically immunodeficiency diseases or autoimmune diseases due to T cell deficiency or reduction associated with congenital dysfunction of the thymus (e.g., congenital thymus hypoplasia or thymic aplasia), acquired impairment or loss of the thymus function, such as those caused by aging, acquired immunodeficiency syndrome caused by infection with human immunodeficiency virus (HIV), and the like. Examples of the immunodeficiency diseases or autoimmune diseases caused by congenital dysfunction of the thymus include pignata guarino syndrome, DiGeorge syndrome, chronic mucocutaneous candidiasis (or APS-1/APECED), and Down syndrome. Examples of congenital immunodeficiency diseases include X-linked lymphoproliferative syndrome, ataxia telangiectasia, hyperimmunoglobulinemia E syndrome, severe combined immunodeficiency, and Wiskott-Aldrich syndrome. Examples of the autoimmune diseases include those listed in the above section "3. Agent for cell transplantation therapy". Examples of cancers to which the immunotherapy is applicable include, but are not limited to, liver cancers (e.g., hepatocellular cancer), ovarian cancers (e.g., ovarian clear-cell carcinoma), childhood cancers, lung cancers (e.g., squamous cell carcinoma and small cell lung cancer), testis cancers (e.g., nonseminomas germ cell tumor), soft tissue tumors (e.g., liposarcoma and malignant fibrous histiocytoma), uterine cancers (e.g., cervical intraepithelial neoplasia and cervical squamous cell carcinoma), melanomas, adrenal tumors (e.g., adenoma of the adrenal gland), neural tumors (e.g., schwannoma), stomach cancers (e.g., adenocarcinoma of the stomach), renal cancers (e.g., Grawitz tumor), breast cancers (e.g., invasive lobular carcinoma and mucinous carcinoma), thyroid cancers (e.g., medullary carcinoma), laryngeal cancers (e.g., squamous cell carcinoma), bladder cancers (e.g., invasive transitional cell carcinoma), and thymomas (including myasthenia gravis).

The T cells of the present invention may be cultured and/or stimulated using a suitable medium and/or stimulating molecules prior to administration to the subject. Examples of the stimulating molecules include, but are not limited to, cytokines, appropriate proteins, antigen-presenting cells, such as dendritic cells, presenting specific antigens on MHCs, peptides derived from specific antigens expected to be presented on MHCs of cells, and other components. Examples of the cytokines include IL-2, IL-7, IL-12, IL-15, and IFN-γ, and of these, IL-2 can be used preferably. The concentration of IL-2 in the medium is not limited to any particular value. For example, the concentration of IL-2 is preferably 0.01 U/mL to 1 × 10⁵ U/mL and more preferably 1 U/mL to 1 × 10⁴ U/mL. Examples of the appropriate proteins include CD3 ligands, CD28 ligands, and anti-IL-4 antibodies. In addition to these, a lymphocyte-stimulating factor such as lectin may also be added. Further, serum or plasma may be added to the medium. The amount of serum or plasma to be added to the medium is not limited to any particular value, and may be, for example, 0 to 20 vol%. Also, the amount of serum or plasma to be used may be changed according to the culturing stage. For example, the concentration of serum or plasma may be reduced stepwise. The serum or plasma may be derived from an autologous or allogeneic source. From the viewpoint of safety, autologous serum or plasma is preferable.

Hematopoietic stem cells or any cells that are in a differentiation process from hematopoietic stem cells to T cells to be used in the present invention can be obtained by known methods. For example, these cells can be obtained by isolating them from biological tissue (e.g., bone marrow, lymph node tissue, thymus tissue, or spleen tissue) or a body fluid (e.g., peripheral blood, cord blood, ascites fluid, or pleural fluid) using a known technique (e.g., isolation using FICOLL) or by inducing differentiation of pluripotent stem cells. From the thus-obtained cell population, desired cells (e.g., CD34 positive cells) may be isolated by flow cytometry.

Hematopoietic stem cells derived from pluripotent stem cells can be produced by, for example, culturing pluripotent stem cells in a base medium supplemented with vitamin Cs. In the present invention, vitamin Cs refer to L-ascorbic acid and derivatives thereof, and derivatives of L-ascorbic acid refer to those that are converted to vitamin C through enzyme reactions *in vivo.* Examples of the derivatives of L-ascorbic acid include vitamin C phosphate, ascorbic acid glucoside, ascorbyl ethyl, vitamin C esters, tetrahexyldecanic acid ascorbyl, ascorbyl stearate, and ascorbyl 2-phosphate 6-palmitate. Of these, vitamin C phosphate is preferable, and examples thereof include salts of L-ascorbic acid phosphate such as L-ascorbic acid phosphate Na and L-ascorbic acid phosphate Mg. The medium used for the production of hematopoietic progenitors may be further supplemented with at least one cytokine selected from the group consisting of MP4 (bone morphogenetic protein 4), VEGF (vascular endothelial growth factor), SCF (stem cell factor), and FLT-3L (Flt3 ligand). Examples of the type of the base medium include those listed in the above section "1. Thymic epithelial cell production method".

The T cells of the present invention may be provided as a cell population in which thymic epithelial progenitors and/or thymic epithelial cells are present, or alternatively, may be provided in the form of an organoid such as a thymic organoid. The term "organoid" as used herein refers to a structure containing two or more types of cells, and the organoid typically has a structure and functions similar to an organ of a living organism. A thymic organoid is typically a structure containing thymic epithelial cells and T cells.

Typically, an ATO culture method can be used to perform the T cell production method of the present invention. The ATO culture method can be performed in the following manner, for example. First, a cell suspension containing thymic epithelial cells together with hematopoietic stem cells or any cells that are in a differentiation process from hematopoietic stem cells to T cells is centrifuged to cause aggregation. A culture vessel containing a T cell induction medium is provided, a membrane (e.g., a membrane made of a polycarbonate film) is placed on the medium, and the aggregates obtained in the above are placed on the membrane. By continuing the culture of the cells in this state, T cells at various differentiation stages can be induced. The T cell induction medium is not limited to any particular medium as long as desired T cells can be induced, and may be, for example, a medium that contains a base medium (e.g., RPMI 1640) containing FLT3L, IL-7, a serum-free B27 supplement, L-ascorbic acid, and a ROCK inhibitor.

Also, T cells may be sorted from a cell population or organoid obtained by the T cell production method of the present invention using the expression of a desired T cell marker (e.g., CD4, CD8, or CD3) as an index. Known methods can be used as appropriate for T cell sorting, and examples of the methods include: labeling cells with an antibody against the T cell marker and sorting the cells by flow cytometry or mass cytometry; magnetic cell separation; and sorting cells using an affinity column on which a desired antigen is immobilized.

As described above, the present invention enables production of T cells with broad reactivity. Such broad reactivity of T cells is achieved because a T cell population with a diverse collection of TCRs (i.e., TCR repertoire) is produced by the present invention. Thus, in one embdoiment, the present invention produces a cell population comprising a plurality of T cells expressing different TCRs. The diversity of TCRs can be evaluated, for example, based on a diversity index. Examples of such a diversity index include the Shannon-Weaver index H' (Shannon index), Inv. Simpson's index 1/λ (Simpson index (reciprocal)), Pielou's evenness (Pielou's index), and DE50 (Diversity Evenness score). As can be seen from Examples to be described below, in one embodiment, the T cell production method of the present invention could produce a cell population exhibiting a Shannon's index of 6.608 for human TCRα and 7.248 for human TCRβ, and the diversity level of the cell population was comparable to that of peripheral blood of healthy individuals.

The time period required for the T cell production method of the present invention is typically from 1 to 6 weeks, preferably 2 to 6 weeks, and more preferably from 5 to 6 weeks, although it may vary depending on the type of desired cells.

Regarding specific culture methods and the like to be used in the T cell production method of the present invention, descriptions on culture methods and the like provided in the above section "1. Thymic epithelial cell production method" are all incorporated in the present section.

The present invention will be more fully understood with reference to the following examples, which provide exemplary non-limiting embodiments of the present invention.

### EXAMPLES

### <Materials and methods>

### Induction of thymic epithelial progenitors from iPS cells

Induction protocol of the present invention comprises the step of inducing FOXN1-expressing thymic epithelial progenitors (TEPs) from human iPS cells in a step-wise manner over the course of 28 days. Four days before the start of the induction, iPS cells cultured in feeder-free conditions were seeded at a density of 1-3 × 10³ cells/cm² to laminin-coated cell culture plates in AK02N medium supplemented with 10 µM Y-27632. On the next day, the medium was changed to AK02N without supplements.

From day 0 of the induction, the base medium was changed to CDM2 and the cells were induced to anterior foregut endoderm (AFE) as described previously (e.g., Loh, K.M. et al., Cell Stem Cell. 2014 Feb 6; 14(2): 237-52). Briefly, CDM2 consists of a 1:1 mixture of IMDM and F12, 1 mg/ml polyvinyl alcohol, 0.7 µg/ml insulin, 15 µg/ml transferrin, 450 µM monothioglycerol, and 1x chemically defined lipids. Primitive streak (PS) was induced through the addition of 100 ng/ml Activin A, 2 µM CHIR99021, and 50 nM PI-103 for one day. Definitive endoderm (DE) was induced from PS by supplementing 250 nM LDN193189 and 100 ng/ml Activin A for two days. AFE was induced from DE by adding 1 µM A83-01 and 250 nM LDN193189 for four days.

After the AFE stage on day 7, 150 nM retinoic acid (RA) (Cas No: 302-79-4) and 50 ng/ml FGF-8 were supplemented to induce pharyngeal endoderm (PE) by day 18. From the PE stage, all supplements were withdrawn and only the base medium CDM2 was used to induce FOXN1-expressing TEP by day 28. TEP were maintained in CDM2 medium and allowed to further mature until at least day 78 to allow undirected differentiation into the cortical and medullary lineages. The medium was changed every 2-3 days.

### Establishment of FOXN1-mCherry reporter lines

Two FOXN1 reporter clones were established from the 201B7 human iPS cell line using the CRISPR/Cas9 system. gRNA sequences were designed to contain a 20 nt protospacer sequence targeting the stop codon and 3' UTR of FOXN1. The pENTR-Donor-MCS2 plasmid vector, containing a puromycin resistance cassette, was used as the backbone to insert the T2A-mCherry sequence and 500-600 bp homology arms corresponding to the C-terminus and the 3' UTR of FOXN1. Cloning was performed with the In-Fusion HD Cloning Kit (Takara Bio, 639648) and DH5a competent cells (Toyobo, DNA-903). Transfection into the 201B7 iPS cell line was performed with the P4 Primary Cell 4D-Nucleofector X Kit S (Lonza, V4XP-4032), using 1 ug of the vector and RNPs formed with 1.25 µg gRNA and 5 µg Cas9 protein.

Three days after transfection, cells were treated with 0.5 µg/ml puromycin to select for clones incorporating the insert. Surviving colonies were picked and replated after one week. DNA was extracted using the NucleoSpin Tissue XS kit (Macherey-Nagel, 740901.50) and PCR was performed using PrimeSTAR GXL DNA Polymerase (Takara Bio, R050A) to confirm the presence of the insert. As the insert was incorporated into only one allele, the other allele was sequenced (Eurofins Genomics) to confirm the absence of indels. mCherry fluorescence was observed using the Keyence BZ-X810 microscope.

### mRNA expression analysis

Cells were dissolved in TRIzol Reagent, the RNA was extracted using the Direct-zol RNA MicroPrep kit (Zymo Research, R2062), and reverse transcription was performed with 300 ng of RNA using the PrimeScript RT reagent Kit with gDNA Eraser (Takara Bio, RR047A). To analyze the mRNA expression, qPCR was performed with the THUNDERBIRD Next SYBR qPCR Mix (Toyobo, QPX-201) using the QuantStudio 12K Flex Real-Time PCR System. All results were adjusted by the ACTB expression in each sample and compared to purified thymic epithelial cells (TEC) from a human neonatal thymus. In the case when mRNA expression was undetected even after 45 cycles of amplification, the relative quantification value was set to zero.

### Flow cytometry

To determine the FOXN1-mCherry⁺ TEP induction efficiency, induced cells were detached using Accumax for 30 min at 37°C, washed in PBS buffer containing 0.1% BSA, and filtered through a 35 µm cell strainer. DAPI was added at 0.5 µg/ml to stain dead cells. Sorting of the cells was performed using the BD FACSAria II flow cytometer, and the FlowJo software was used to analyze the results.

### Preparation of heat map

Expression levels of the respective genes were measured by qPCR in the iPS cells, primary TECs, and cells sorted on days 28, 39, 57, and 80 of culture by flow cytometry using mCherry expression as an index (mCherrry^{low} population and mCherry^{high} population). Then, a heat map was created with Microsoft Excel using the expression levels in the primary TECs as reference expression levels.

### Single cell RNA sequencing

For single cell RNA sequencing, induced cells were processed and sorted as described in the Flow cytometry section to enrich for mCherry⁺ cells. 16000 cells were loaded onto chips of the Next GEM Chip K kit (10x Genomics, 1000287) and the cell emulsion was created using the Next GEM Single Cell 5' GEM Kit v2 (10x Genomics, 1000244) and the Next GEM Single Cell 5' Gel Bead Kit v2 (10x Genomics, 1000264), after which the RNA was reverse transcribed and purified according to the manufacturer's instructions. The library was constructed using the Library Construction Kit (10x Genomics, 1000190) and sequenced using the Illumina NovaSeq 6000 at 50000 reads per cell.

The sequencing data were processed using the Cell Ranger pipeline and analyzed using the Seurat 4.0 package in R. QC was performed by including only cells with <10% mitochondrial counts, unique feature counts between 2500 and 6500, and unique RNA counts between 5000 and 30000. The data were normalized and scaled, and PCA was performed. Dimensional reduction was performed using tSNE and clusters were analyzed for marker genes of different cell types.

### Immunostaining

On day 80 of the TEC induction, the induced cells were immobilized using 4% paraformaldehyde. After washing the cells with PBS, the cells were permeabilized using 0.4% Triton X-100 and then washed with PBST. The cells were blocked with 1% BSA blocking buffer in PBS for 1 hour at room temperature and stained overnight at 4°C in the same blocking buffer using a primary antibody against EPCAM (BioLegend) at 1:600, a primary antibody against HLA-DR (BioLegend) at 1:600, a primary antibody against PSMB11 (Proteintech) at 1:600, a primary antibody against KRT5 (BioLegend) at 1:600, and a primary antibody against mCherry (abcam) at 1: 1500. On the next day, the cells were washed with PBST and stained with antimouse and anti-rabbit antibodies having Alexa Fluor 488 conjugated thereto and anti-chicken antibody having Alexa Fluor 594 conjugated thereto (all Thermo Fisher) in the blocking buffer for 1 hour at room temperature. At the same time with the use of the secondary antibodies, DAPI (Dojindo) was used at 1:5000 to counterstain the nuclei. The stained cells were washed with PBST and then stored in PBS at 4°C until observation under the Keyence BZ-X810 microscope.

### Induction of differentiation of human thymic CD4⁻CD8α⁻ DN T cells into SP T cells using iTEPs

### 1. Collection of FOXN1⁺ iTECs

On days 30 to 35 following the induction of iTEPs from FOXN1-mCherry reporter iPS cells, the cells were washed once with PBS and incubated with Accumax for 30 minutes at 37°C in 5% CO₂. Further, the cells were detached using a cell scraper, and the cell suspension was diluted 10-fold with PBS + 1% FBS and centrifuged at 1300 rpm for 5 minutes. After the centrifugation, the supernatant was removed, and the cells were resuspended in PBS + 1% FBS. The resulting cell suspension was filtered and transferred to a 5 ml FACS tube. DAPI was added to the cell suspension at 0.1 µg/ml, from which mCherry⁺ cells were sorted using BD FACS Aria^{™} II & III cell sorters.

### 2. Collection of human thymic T cell progenitors (CD4/8 double-negative (DN) T cells)

Human thymic tissue was fragmented, and the thus-obtained fragments were added to RPMI 1640 + 1% FBS and vortexed. Thereafter, a supernatant containing thymocytes were collected. Vortexing was repeated until the medium turned into a clear colorless solution, and the collected supernatant was centrifuged at 1300 rpm for 5 minutes. After the centrifugation, the supernatant was removed, and the residue was treated with 1x RBC Lysis Buffer at 4°C for 5 to 10 minutes to cause hemolysis. The Lysis Buffer was diluted 5-fold with RPMI 1640 + 1% FBS and centrifuged at 1300 rpm for 5 minutes. The supernatant was removed, and the thymocytes were resuspended in PBS + 1% FBS. Antibodies (CD11c, CD1, CD19, CD8α, CD4, CD5, CD7, and CD45) were added to the thymocyte suspension at 1:20, and the resulting mixture was incubated at 4°C in the dark for 20 minutes. The stained cells were washed with PBS + 1% FBS and centrifuged at 1300 rpm for 5 minutes. After the centrifugation, the supernatant was removed, and the cells were resuspended in PBS + 1% FBS. The resulting cell suspension was filtered and transferred to a 5 ml FACS tube. PI (Propidium Iodide) was added to the cell suspension at 1:500, from which CD11c⁻CD14⁻CD19⁻CD5⁺CD7⁺CD8⁻CD4⁻ cells (DN T cells) were sorted using the BD FACSAria^{™} II & III cell sorters.

### 3. Differentiation culture of T cells by co-culture of iTECs and T cell progenitors

The mCherry⁺ cells and DN T cells obtained after sorting were mixed together at a ratio of 1 × 10⁵:5 × 10³ to 1 × 10⁴, and the resulting mixture was centrifuged at 1300 rpm for 5 minutes. The supernatant was removed, and cell pellets were resuspended using 2.5 to 5 µl of Matrigel. An induction medium (RPMI 1640 + 4% RB27 + 5 ng/ml rhFLT3L + 5 ng/ml rhIL-7 + 30 µM L-ascorbic acid + Rock inhibitor) was added to a 12-well plate at 1 ml/well, and Nuclepore Track-Etched membranes (Whatman 13MM 0.8 µm PVPF) were placed on the medium, and the above-described cell suspension was placed on the membranes. The medium was changed every 3-4 days.

### 4. Confirmation of T cell differentiation

### • Day 14 (time at which CD4/8 double-positive (DP) cells appear)

Organoids were crushed and centrifuged at 1300 rpm for 5 minutes. Antibodies (CD3, CD8α, CD4, TCRγδ, TCRαβ, and CD45) were added to the suspension at 1:20, and the resulting mixture was incubated at 4°C in the dark for 20 minutes. The stained cells were washed with PBS + 1% FBS and centrifuged at 1300 rpm for 5 minutes. After the centrifugation, the supernatant was removed, and the cells were resuspended in PBS + 1% FBS. The resulting cell suspension was filtered and transferred to a 5 ml FACS tube. PI was added to the cell suspension at 1:500, and CD4⁺CD8⁺ DP T cells were observed using the BD FACSAria^{™} II & III.

### • Day 34 (time at which CD4 or CD8 single-positive (SP) cells appear)

Organoids were crushed and centrifuged at 1300 rpm for 5 minutes. Antibodies (CD3, CD8α, CD4, TCRγδ, TCRαβ, CD45RA, CD8β, CD62L, CCR7, and CD27) were added to the suspension at 1:20, and the resulting mixture was incubated at 4°C in the dark for 20 minutes. The stained cells were washed with PBS + 1% FBS and centrifuged at 1300 rpm for 5 minutes. After the centrifugation, the supernatant was removed, and the cells were resuspended in PBS + 1% FBS. The resulting cell suspension was filtered and transferred to a 5 ml FACS tube. PI was added to the cell suspension at 1:500, and CD8αβ SP T cells (CD3⁺TCRαβ⁺CD8α⁺CD8β), naive CD8⁺ SP T cells (CD3⁺TCRαβ⁺CD8α⁺CD45RA⁺CD62L⁺CCR7⁺CD27⁺), and naive CD4⁺ SP T cells (CD3⁺TCRαδ⁺CD4⁺CD45RA⁺CD62L⁺CCR7⁺CD27⁺) were observed using the BD FACSAria^{™} II & III cell sorters.

### Example 1: Induction of differentiation from iPS cells to TECs

### Increase in expression of marker genes in respective developmental stages in induction process to TEPs (FOXN1-expressing cells)

The expression of marker genes at respective differentiation stages (AFE, PE, and TEP) was examined over time through qPCR using iPS cells as a negative control and primary TECs collected from the human thymus as a positive control, and the expression levels of these marker genes were plotted on a graph as ratios relative to those in the primary TECs collected from the human thymus. The expression of AFE markers started to increase at around day 7 (D7) after culturing and the expression of PE markers started to increase at around day 18 (D18) after culturing. Further, by day 28 (D28) after culturing, the expression level of FOXN1, which is a TEP marker, also increased to about half the expression level thereof in the primary TECs (FIG. 1). Since it is presumed that not all the cultured cells were FOXN1 positive, the expression level of FOXN1 per cell is considered to be equivalent to or higher than that in the primary TEC.

### Confirmation of FOXN1 expression using FOXN1 reporter iPS cells

### a) Vector used for establishment of FOXN1-mCherry reporter iPS cell line

A vector was designed so as to allow independent expression of mCherry accompanying the expression of FOXN1, and the vector was transfected into iPS cells (201B7) through genome editing to obtain positive clones (FIG. 2-a).

### b) Increase in expression of FOXN1-mCherry during culture

The expression levels of mCherry on days 28, 35, and 42 after culturing are shown. It can be seen that the number of mCherry positive cells increased in keeping with an increase in the number of days in culture (FIG. 2-b).

### c) Comparison of FOXN1 expression level with that in TECs isolated from human thymus (primary TECs)

mCherry positive cells on day 45 were collected using a cell sorter, and the expression level of FOXN1 in the mCherry positive cells was compared with that in primary TECs by qPCR. The result revealed that the mCherry positive cells obtained through the above-described induction process expressed FOXN1 at a level equal to or higher than that in the primary TECs (FIG. 2-c).

### Increase in expression of TEC maturation/functional markers and cortical/medullary markers in late stage of culture (from day 45 onward)

The present experiment examined the possibility that maturation of TECs or differentiation of TECs into cell lineages with different functions would be induced. More specifically, the expression of TEC maturation/functional marker and cortical/medullary marker genes in the cells after the FOXN1 expression had been confirmed (from D45 onward) was examined by qPCR in the same manner as described above in connection with FIG. 1, and their expression levels were plotted on a graph as ratios relative to those in the primary TECs.

### a) Decrease in expression of PE marker (TBX1) and parathyroid marker (GCM2) of closely related cell lineage and increase in expression of FOXN1 and TEC maturation marker (HLA-DRA)

Expression of an undifferentiation marker (TBX1) and a parathyroid marker (GCM2) of a closely related cell lineage started to markedly decrease after D65, and a further increase in the expression of FOXN1 and an increase in the expression of MHC class II molecules (HLA-DRA), which is the most important TEC maturation marker, were observed (FIG. 3-a).

### b) Increase in expression of functional molecules of TECs

Cytokines (IL7, KITLG), chemokines (CXCL12, CCL25), and NOTCH ligand (DLL4), which all support T cell production, were expressed at levels equivalent to or higher than those in the primary TECs.

### c) Increase in expression of cortical thymic/medullary thymic epithelial markers

The expression of cortical epithelial markers (KRT8, PSMB11, and PRSS16) and medullary epithelial markers (KRT5, RANK, CD40, and AIRE) was examined. The expression levels of the marker genes of the cortical epithelial cells were generally higher than those in the primary TECs (FIG. 3-c). On the other hand, the expression levels of the medullary epithelial markers tended to be lower than those in the primary TECs (FIG. 3-c). Based on previous studies using mouse models, it has been considered that differentiation into cortical epithelial cells is default and the cortical epithelial cells differentiate into the medullary epithelium by receiving further signals, and the above results are consistent with this consideration. Signals mediated by RANK or CD40 promote the proliferation and differentiation of the medullary epithelium, in particular, the differentiation of AIRE-expressing cells. Accordingly, supplementing the present culture system with RANKL or CD40 L may lead to differentiation into the medullary epithelium further including AIRE-expressing cells, which contribute to the establishment of self-tolerance.

### Confirmation of differentiation of mature medullary thymic/cortical thymic epithelial cells by single-cell analysis

### a) Expression of FOXN1/mCherry and epithelial marker (EPCAM) and maturation marker (HLA-DR)

(Left) Cells on day 78 were dissociated into single cells. mCherry positive (P3) and mCherry negative (P4) fractions were obtained by sorting, and they were mixed together at a ratio of 4:1 for single-cell analysis. Different cell clusters were identified based on the difference in gene expression (FIG. 4-a).

(Right) The expression of FOXN and mCherry was observed in the majority of cells. It can be seen that the expression levels thereof were particularly high in cell populations closer to the right from the center (FIG. 4-a).

### b) Expression of cortical thymic epithelial markers

Expression distribution of the cortical epithelial markers was examined. The expression levels thereof were high in cell populations closer to the right from the center, in which FOXN1 was highly expressed (FIG. 4-b). This result is consistent with previous findings that the mature thymus exhibits high FOXN1 expression at its cortical epithelium and also consistent with the results seen in the atlas constructed by human thymus scRNAseq, which can be found in a public database (https://developmentcellatlas.ncl.ac.uk/datasets/HCA_thymus/).

### c) Expression of medullary thymic epithelial markers

Medullary markers were examined. In consistent with the qPCR results, the expression levels thereof were lower and the number of cells expressing them was smaller as compared to those of the cortical epithelial markers. However, cell populations preferentially expressing the medullary epithelial markers were observed in cell clusters generally closer to the left from the center (FIG. 4-c). These results are also consistent with previous findings that FOXN1 expression is low in medullary epithelium, especially in mature medullary epithelium.

### d) Confirmation of differentiation into a plurality of subsets of thymic epithelium

Cell populations were annotated based on the expression of the above-described major markers (FIG. 4-d). The names of the subsets were determined with reference to the human scRNAseq atlas described in b) above.

### Example 2: Analysis of expression patterns of genes of respective cells in differentiation induction from iPS cells to TECs

In the late stage of TEC induction, separation of a mCherry⁺ population into two different populations, namely, mCherry^{low} population and mCherry^{high} population, was often observed through flow cytometry. Thus, both cell populations were sorted separately and tested for expression of cortical thymic epithelial cell (cTEC) markers and medullary thymic epithelial cell (mTEC) markers using qPCR. The results are shown in FIG. 5. A pharyngeal endoderm (PE) marker TBX1 and an early parathyroid (PT) marker GCM2 were highly expressed in both cell populations at day 28, but the expression levels thereof dropped sharply to be equal to the levels thereof in the primary TECs (in the mCherry^{high} population) or to be nearly equal to the levels thereof in the primary TECs (in the mCherry^{low} population). The expression level of FOXN1, which is a master regulator of TECs, was similar to that in the primary TECs in the mCherry^{low} population and was higher than that in the primary TECs in the mCherry^{high} population. The cTEC markers were highly expressed only in the mCherry^{high} population and often 3 to 10 times higher than those in the primary TECs, whereas the mTEC markers were observed at lower expression levels than in the primary TECs in both populations. In particular, the expression level of KRT5, which is an early mTEC marker, was higher than that in the primary TECs in the mCherry^{low} population, whereas the expression level of KRT5 was much lower than that in the primary TECs in the mCherry^{high} population. On the other hand, markers of more mature mTEC subpopulations, including CCL19 and CCL21, were equally observed regardless of the mCherry expression level. The majority of the tested cTEC and mTEC markers exhibited increased expression during the induction period (HLA-DRA exhibited the most notable increase), and this suggests that the induced TECs continue to mature at least until day 80 and possibly beyond. Taken together with the fact that higher FOXN1 expression in cTECs than in mTECs has been reported for primary TECs (Campinoti S. et al. (2020), Nat. Commun., 11(1): 6372), these results suggest that the mCherry^{high} population was predominantly made up of mature cTECs and contained few mTECs, whereas the mCherry^{low} population contained a mixture of immature mTECs and possibly bipotent progenitors.

The results of immunostaining are shown in FIG. 6. The results of immunostaining showed that, on day 80 post-induction, EPCAM, which is an epithelial marker, was observed in most of the cells present inside and outside the mCherry⁺ colony, whereas HLA-DR, which is a mature TEC marker, was detected almost exclusively in the mCherry+ colony. PSMB11, which is a highly specific mature cTEC marker, was observed only in mCherry⁺ cells, and almost all of these cells were PSMB11 positive. KRT5, which is an early mTEC marker, was observed both inside and outside the mCherry⁺ colony. In summary, these results suggest that most of the mCherry⁺ cells detected by this immunostaining correspond to the mCherry^{high} cells observed by the flow cytometry, whereas other regions may contain both mCherry⁻ cells and mCherry^{low} cells that are too dark to be reliably detected by this method. The high frequency of PSMB11 and HLA-DR in the mCherry⁺ colony suggests that these cells were mature cTECs, and this confirms the qPCR results. The presence of KRT5 both inside and outside the mCherry⁺ colony indicates that immature mTECs may be found in both mCherry^{high} population and mCherry^{low} population. Similar results were observed in three iPSC lines (201B7, 409B2, and 1383D6), and this demonstrates the robustness of this induction system to reliably induce TECs from iPSCs.

### Example 3: Induction of T cells from hematopoietic stem cells

EpCAM⁺mCherry⁺ iTEPs on days 30 to 35 post-induction were co-cultured with human thymus-derived T cell progenitors (CD4/8 DN cells). The results are shown in FIG. 7. Differentiation into CD4/8 DP cells was observed in about 2 weeks after culturing the cells. The proportion of CD4/8 DP was approximately equivalent to that in the positive control in which human DLL1 (MS5/hDLL1) was used in mouse stromal cells MS5 (expressing only mouse class I). Differentiation into DP was not observed when the EpCAM⁺mCherry⁻ fraction was used.

In about 4 weeks after co-culturing with the EpCAM⁺mCherry⁺ iTEPs, induction of TCRαβ⁺ CD4 or CD8 positive SP T cells was observed. The iTEPs expressed human MHC class I/II molecules at protein levels, and this suggests that the cells had differentiated into CD4 CD8 SP cells restricted to human MHC. Since few T cell progenitors remained in the culture medium when the EpCAM⁺mCherry⁻ fraction was used, it is considered that this fraction had very low activity in maintaining and supporting the differentiation of T cell progenitors. In co-culture with the MS5/hDLL cells, although induction of CD8 SP was observed as reported previously (considered to be restricted to mouse MHC class I), it was found that the induction efficiency of CD4 SP was very low. In the co-culture with MS5, few T cell progenitors remained in the culture medium.

### Example 4: Repertoire analysis

Repertoire analysis was outsourced to Repertoire Genesis Inc. A specimen (specimen name: CD8Sortingcell, the amount of RNA solution: 20.0 µL, and nanodrop: 12.6 ng/µL) was obtained by co-culturing EpCAM⁺mCherry⁺ iTEPs and human thymus-derived T cell progenitors (CD4/8 DN cells) in the same manner as in Example 3 and sorting cells in a CD4⁺CD8α⁺ fraction (the lower right fraction in FIG. 8) on day 34 after culturing. The cells were sorted from about 30 organoids, and 10000 of them were subjected to the analysis. The outline of the test conducted for the analysis is as follows.
Title of test: Repertoire analysis of three-dimensional co-cultured CD8⁺ T cells
Test number: 23-020-S
Analyte: Total RNA
Test item: Human TCR alpha, beta repertoire analysis
Number of specimens: 1
Test method: By TCR/BCR repertoire analysis (T-0001)
Analysis software: Repertoire Genesis (Software Ver. 20180912, Database Ver. 20180912)
Date of receipt of application: March 16, 2023
Date of test: March 23, 2023 to April 17, 2023

FIG. 9 shows the results for human TCRα (TRA), and FIG. 10 shows the results for human TCRβ (TRB). Among various diversity indices, the Shannon-Weaver index H' is particularly important, and the values of this index indicate that the diversity levels of both TRA and TRB are generally comparable to those of peripheral blood of healthy individuals.

The present application claims priority based on U.S. Provisional Patent Application No. 63/353,330 (filing date: June 17, 2022), the disclosure of which is incorporated herein in its entirety by reference.

## Claims

1. A method for producing thymic epithelial cells, comprising culturing thymic epithelial progenitors for a long period of time.

2. The method according to claim 1, wherein the thymic epithelial progenitors are cultured for at least 25 days.

3. The method according to claim 1 or 2, wherein the thymic epithelial progenitors are cultured in the absence of at least retinoic acid and FGF-8.

4. The method according to any one of claims 1 to 3, wherein the thymic epithelial progenitors are cultured in the absence of at least one factor selected from the group consisting of sonic hedgehog, BMP-4, and noggin.

5. The method according to any one of claims 1 to 4, wherein the thymic epithelial progenitors are obtained by culturing a pharyngeal endoderm cell in the absence of at least retinoic acid and FGF-8.

6. The method according to claim 5, wherein the pharyngeal endoderm cells are obtained by culturing anterior foregut endoderm cells in the presence of retinoic acid.

7. The method according to claim 6, wherein the culture is performed in the presence of FGF-8 in addition to the retinoic acid.

8. The method according to any one of claims 1 to 7, wherein the culture is performed under feeder-free and/or xeno-free conditions.

9. The method according to any one of claims 1 to 8, comprising sorting cells using an expression intensity of FOXN1 as an index.

10. A method for producing T cells, comprising bringing thymic epithelial cells obtainable by the method according to any one of claims 1 to 9 into contact with hematopoietic stem cells or with any cells that are in a differentiation process from hematopoietic stem cells to T cells.

11. The method according to claim 10, wherein the thymic epithelial cells and the hematopoietic stem cells are derived from the same individual.

12. The method according to claim 10 or 11, wherein the T cells are selected from the group consisting of CD4 and CD8 double-positive cells, CD4 positive cells, and CD8 positive cells.

13. A method for producing thymic epithelial progenitors, comprising:
(i) culturing anterior foregut endoderm cells in the presence of retinoic acid to induce differentiation into pharyngeal endoderm cells; and
(ii) culturing the pharyngeal endoderm cells in the absence of at least retinoic acid and FGF-8 to induce differentiation into thymic epithelial progenitors.

14. The method according to claim 13, wherein the culture in the step (i) is performed in the presence of FGF-8 in addition to the retinoic acid.

15. Thymic epithelial cells obtainable by the method according to any one of claims 1 to 9, T cells obtainable by the method according to any one of claims 10 to 12, or thymic epithelial progenitors obtainable by the method according to claim 13 or 14.

16. An agent for transplantation therapy, comprising the cells according to claim 15.
